(19) Europäisches Patentamt

European Patent Office

Office européen des brevets

(11) **EP 0 917 078 A1**

(12) **EUROPEAN PATENT APPLICATION**

(43) Date of publication:
**19.05.1999 Bulletin 1999/20**

(51) Int. Cl.⁶: $G06F\ 19/00$, $G06F\ 9/44$

(21) Application number: **97308240.7**

(22) Date of filing: **14.10.1997**

(84) Designated Contracting States:
**AT BE CH DE DK ES FI FR GB GR IE IT LI LU MC NL PT SE**
Designated Extension States:
**AL LT LV RO SI**

(71) Applicant:
**SMITHKLINE BEECHAM CORPORATION**
**Philadelphia Pennsylvania 19103 (US)**

(72) Inventors:
• **Boyko, David Alexander**
  **Philadelphia, Pennsylvania 19103 (US)**
• **Gallo, Edward Francis**
  **Philadelphia, Pennsylvania 19103 (US)**

• **Langer, Dennis**
  **Philadelphia, Pennsylvania 19103 (US)**
• **Press, Bruce**
  **Philadelphia, Pennsylvania 19103 (US)**
• **Stavrakas, Spyros**
  **Philadelphia, Pennsylvania 19103 (US)**
• **On Wong, Bruce Jan**
  **Philadelphia, Pennsylvania 19103 (US)**

(74) Representative:
**Thompson, Clive Beresford et al**
**SmithKline Beecham plc**
**Corporate Intellectual Property,**
**Two New Horizons Court**
**Brentford, Middlesex TW8 9EP (GB)**

(54) **Disease management method and system**

(57)     The Disease Management system and method includes a Patient Medical Information source (100), a Predictive Health Outcome Modeling process (102), a process for Intervention of At-risk Patients (103), and a source of disease management Modeling Guidelines (104). The Patient Medical Information source (101) is a database containing medical records of patients who participate in a healthcare provider's program. The Predictive Health Outcome Modeling process (102) produces a statistical model used to predict whether a patient with a particular disease is likely to suffer an adverse health outcome. The Intervention of At-risk Patients process (103) derives a list of at-risk patients who have a high risk of suffering an adverse health outcome and intervenes in the selected patient's healthcare treatment to decrease the possibility of such an adverse health outcome. The Predictive Health Outcome Modelling process (102) 1) receives a sample group of patient medical data from the Patient Medical Information database (100) for a given disease, 2) receives pre-determined statistical information for generating predictive models, shown as the Modeling Guidelines (104), and 3) generates a particular predictive model for a particular disease to determine the probability of an adverse health outcome. The Intervention of At-risk Patients process (103) 1) receives the predictive model provided by the Predictive Health Outcome Modeling process (102), 2) analyzes the individual patient specific medical data from the Patient Medical Information database (100), and 3) identifies a list of current patients that are at-risk of an adverse health outcome for a particular disease. The Intervention of At-risk Patients (103) process intervenes in the treatment process of the patients contained in the patient list through contact with the patient, physician, or healthcare provider, and the process requires externally generated information about treatment regimens for given stages of disease progression, as well as particular interventions.

FIG. IA

**Description**

FIELD OF THE INVENTION

This invention relates to electronic computational processing techniques in the field of human healthcare and, more particularly, to identification of high-risk patients for disease and disease intervention management using various electronic computational processing techniques.

BACKGROUND OF THE INVENTION

Diseases or condition can be more effectively and more cost-effectively treated by designing a program to maximize compliance with current best medical practices which are also consistent with a given preferred treatment regimen and on a case by case basis. Treatment for many types of diseases has moved from episodic, symptomatic treatment to disease reduction and prevention.

Healthcare costs in general are rising rapidly, and, in many cases, the costs of treating patients is not distributed evenly among the total population of patients because it costs more to treat some patients than others. This is partly due to some patients not receiving appropriate therapies for their medical condition. This problem has several causes, including that some patients do not comply with their prescribed treatment regimens, that some patients do not visit their doctors at appropriate times, and, in some cases, that some doctors are not aware that a certain therapy regimen is more likely to be more effective than their current regimen.

If patients are treated in accordance with therapy regimens proven to be effective for a given state of disease progression, then the total costs of treating the whole population will decline. If more patients are treated properly, then the number of cases which progress to more serious stages of disease, which are more costly to treat, will he reduced.

SUMMARY OF THE INVENTION

The present invention is a computer-implemented system and method for identifying at-risk patients, particularly those diagnosed with an identified disease, where the information about patients is extracted from at least one preexisting in at least one database. The system includes a means for processing the patient information in the database based on a predetermined criteria to extract relevant information for a group of patients having or who may develop the identified disease. The system defines a predictive model, including associated rules, by:

> a) processing, based on predetermined criteria, the patient information in the database to extract patient information for a group of patients relating to an identified disease or condition;
> b) defining a predictive model, including:
>
> > i) defining, using the information available in the database, a set of events or data relevant to the identified disease or condition;
> > ii) converting the extracted patient information and the defined events or data into files comprising event-level information;
> > iii) defining a time-window for providing a timeframe from which to judge whether specific ones of the defined events should be considered in subsequent processing;
> > iv) identifying a set of variables as potential predictors;
> > v) processing the event-level information, using the time-window and the set of variables, to generate an analysis file;
> > vi) performing statistical analysis on the analysis file to generate the prediction model and a set of rules for use in identifying at-risk patients diagnosed with or who may develop the identified disease or condition, said prediction model and rules being a function of a subset of the set of variables;
>
> c) applying the prediction model and the rules to the same or new set of event-level information to identify at-risk patients for the identified disease or condition, or to identify patients who may be at risk for developing the identified disease or condition;
> d) preparing an intervention list from the identified at-risk patients and selecting, for at least one at risk patient, an intervention;
> e) distributing or facilitating the distribution of the intervention to said patient; and optionallyf) recording and tracking an intervention result for each at-risk patient based on the respective selected intervention; and optionally
> g) updating the historical data in at least one database with each intervention result corresponding to said database; and

repeating step b(ii), and

h) re-applying the prediction model and rules to the event-level information extracted from the data in the updated database.

BRIEF DESCRIPTION OF THE DRAWINGS

The invention is best understood from the following detailed description when read in connection with the accompanying drawing, in which:

Figure 1A is a high level diagram of the Disease Management System of the present invention

Figure 1B is a high level flowchart illustrating an exemplary overall process of the Disease Management System of the present invention.

Figure 2A is a high level flowchart illustrating the raw patient data acquisition, pre-processing, and database formation of the present invention.

Figure 2B is a high-level block diagram illustrating three exemplary sources of information suitable for use with the present invention.

Figure 3 is a flowchart of an embodiment of the conversion process of the Raw Patient Data Pre-processing process of the present invention.

Figure 4 is an illustration of an exemplary Data Model as used in the Disease Management database of an embodiment of the present invention.

Figure 5 is a diagram illustrating the research database format for each of the Rx, DR, and HL claims of the records contained in the research database of an exemplary embodiment of the present invention.

Figure 6 is a high level flowchart illustrating the Extraction process and Predictive Modeling process for an identified disease of the present invention.

Figure 7A is a diagram illustrating an event level file of one embodiment of the present invention generated for depression as the identified disease.

Figure 7B is a diagram illustrating an event level file of one embodiment of the present invention generated for congestive heart failure as the identified disease.

Figure 8 is a diagram illustrating the format of the analysis file of one embodiment of the present invention for an identified disease.

Figure 9 is a time-line diagram showing the events and prediction window scheme as used in the present invention.

Figure 10A is a time-line diagram which shows a first exemplary time window scheme suitable for use in processing the data from the event level files shown in Figure 7A and Figure 7B.

Figure 10B is a time-line diagram which shows a second exemplary time window scheme suitable for use in processing the data from the event level files shown in Figure 7A and Figure 7B.

Figure 11 is a high level flowchart showing the Risk Stratification process of the present invention including the Front End process and the Mining Engine process to generate an intervention list for an identified disease.

Figure 12 is a high level flow chart showing the Mining Engine of the Risk Stratification process of the present invention.

Figure 13 is a high level diagram of the Intervention Management process of the present invention.

DETAILED DESCRIPTION OF THE INVENTION

**General Overview**

The Disease Management system and method of the invention increases the number of patients within a given population who receive, comply with, and correctly administer appropriate therapies for treating a disease or condition. The invention requires identifying preferred treatment regimens for given stages of disease progression. These regimens may be published medical guidelines or guidelines developed by healthcare professionals for a given type of disease. These guidelines are called Best Practice Guidelines.

The term "disease management" applies to, for example, managed care organization, medical group, employer, or government sponsored programs that identify individual patients with chronic long term conditions that may be at risk of expensive hospitalization or other high cost events or adverse health outcome. Disease management services are defined by a research area in conjunction with product development managers who serve as disease subject matter experts.

Disease management services are offered to clients (participating managed care organizations (MCOs) or other types of subscribers) for the purposes of early intervention at specific disease states to improve future disease outcomes. An individual's medical, clinical and administrative medical history information is provided from, for example, third party processors to the disease management system.

This specification primarily describes use of the Disease Management system of the present invention with regard to the healthcare field in which healthcare providers are the primary clients of the system, and information about the patients of these healthcare providers are provided to a database for the practice of the invention. However, it is contemplated that other embodiments of the invention include other types of clients, such as employers, government agencies, insurance providers, or other users who are interested in disease management or the thriftiness of a given population of individuals. Similarly, the information provided to the database of the Disease Management system could be expanded to include demographic data, socialization, geographic data, family history, or other information about an individual.

A basic disease management system will look at one disease or condition. But multiple diseases or conditions can be factored into a single analysis and thereby develop a risk profile based on multiple factors and multiple diseases or conditions. In essence, the approach is to view each disease or condition as a module which can be cross-referenced like the fields of a relational data base. That permits the analyst to draw on more than one disease or condition in developing a risk factor for a given patient population.

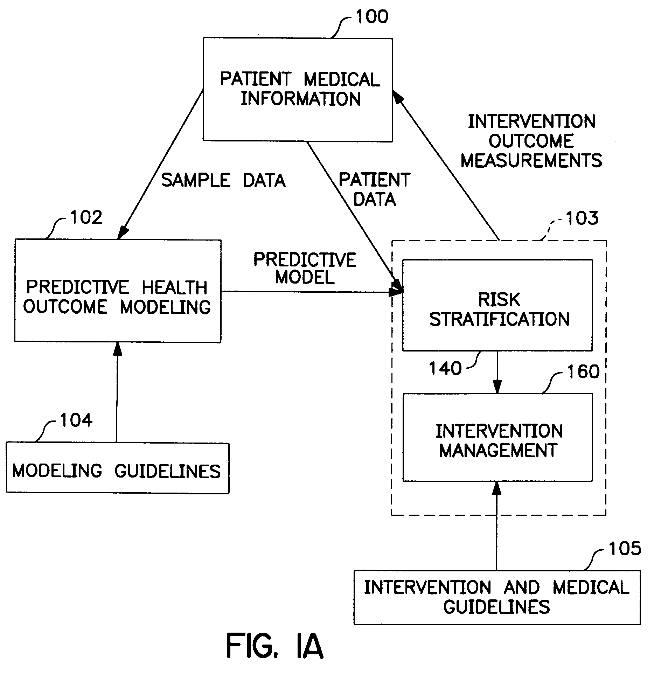

Referring to Figure 1A, the high level diagram of the disease management process of the present invention includes a Patient Medical Information source 100, a Predictive Health Outcome Modeling process 102, a process for Intervention of At-risk Patients 103, a source of disease management Modeling Guidelines 104, and a source of Intervention and Medical Guidelines 105. The process for Intervention of At-Risk Patients 103 has two parts: a Risk Stratification process 140 and an Intervention Management process 160. The Patient Medical Information source 101 is typically a form of database containing, for example, records of medical history, physical descriptions, psychiatric records, laboratory tests results, cognition and intelligence test data, prescriptions and treatment of patients who participate in a healthcare provider's program.

The Predictive Health Outcome Modeling process 102 of Figure 1A is a process that produces a statistical model which can be used to predict whether a patient with a particular disease or condition and medical history is likely to suffer an adverse health outcome. The process of Intervention of At-risk Patients 103 includes: the Risk Stratification process 140 that is a database analysis process which derives a list of at-risk patients who have a high risk of suffering an adverse health outcome, and the Intervention Management process 160 that determines an intervention in the selected patient's healthcare treatment to decrease the possibility of such an adverse health outcome.

The operation of the disease management process of the present invention, as shown in Figure 1A, is now described. First, the Predictive Health Outcome Modeling process 102 receives a sample group of patient data from the Patient Medical Information database 100 for a given disease. In addition, the Predictive Health Outcome Modeling process 102 receives certain pre-determined statistical or other information for generating predictive models, shown as the Modeling Guidelines 104 in Figure 1A, and generates a particular predictive model for a particular disease to determine the probability of an adverse health outcome. The same or similar data could be used to determine the probability of developing a particular disease or condition which is associated with an adverse health outcome.

The Risk Stratification process 140 receives the predictive model provided by the Predictive Health Outcome Modeling process 102 and analyzes the individual patient-specific data from the Patient Medical Information database 100 with the predictive model to identify a list of current patients that are at-risk of an adverse health outcome for a particular disease. Once the list of patients is identified, the Intervention Management process 160 suggests an intervention in the treatment process of the patient through contact with the patient, physician, or healthcare provider. The process of Intervention of At-risk Patients 103 requires externally generated information about treatment regimens (e.g. Best Practice Guidelines) for given stages of disease progression, as well as particular interventions, which are shown as the Intervention and Medical Guidelines 105 of Figure 1A.

Finally, the interventions itself may be recorded, and once the process of Intervention of At-risk Patients 103 has been completed, the results of these interventions, shown as intervention outcome measurements in Figure 1A, are recorded in the Patient Medical Information database 100. This allows for a feed-back step where data after intervention can be fed back through the whole process, either to be again re-run through the Risk Stratification step to help analyze the outcomes or to become part of the basis for generating a new and revised Risk Stratification process.

To summarize, the disease management system analyzes the flow of individual, patient-specific health information and intervenes with the physician or patient whenever necessary to attempt to avoid adverse health outcomes and consequent high cost events. Disease management includes:

1) Identifying the client organization and prospective program patient enrollees based at certain predetermined disease states derived from research data.
2) Utilizing medical claim, pharmacy claim, clinical data and laboratory data to assess disease states.
3) Utilizing pre-defined interventions to manage the program. Examples of interventions could be mailing periodic notifications, mailing disease educational material, patient-initiated phone survey responses or even outbound calling performed by a staff of health care professionals.

4) Administering the process with case program managers who perform the necessary intervention with the client (e.g. MCO, healthcare provider), doctor and patient.

5) Recording interventions in patient care to determine if proactive disease management services improve specific disease outcomes.

6) Processing intervention management information back through an analytic process to determine the outcome of intervention.

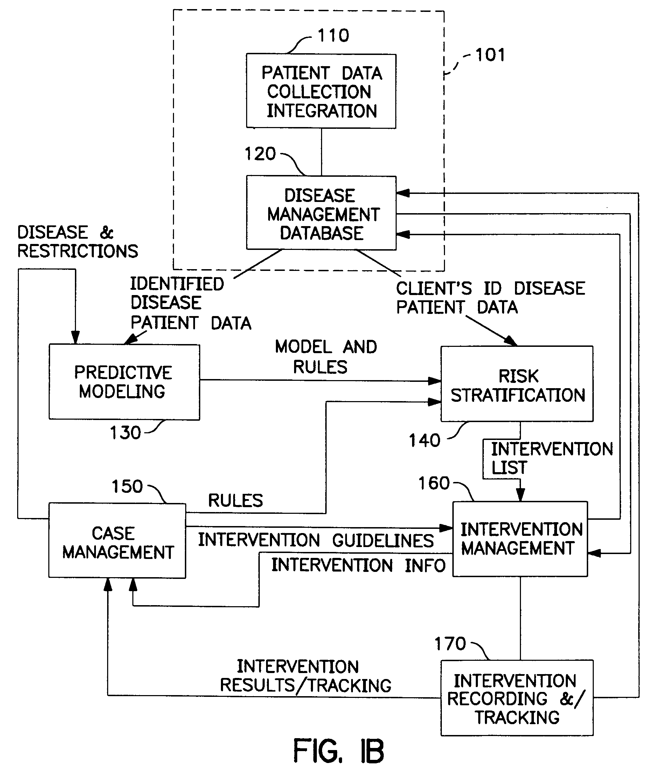

In the method of this invention, the case program manager (not shown in Figure 1A, but whose functions are shown as part of the Case Management process 150 of Figure 1B, which is described in detail below) facilitates patient treatment by identifying to physicians patients who are likely to benefit from a change in therapy; and by suggesting therapies to the physicians; and by providing educational and treatment compliance assistance to patients (with the concurrence of the treating physicians). The case program manager does not diagnose disease or prescribe treatment regimens. Medical diagnosis and treatment is the sole responsibility of licensed physicians.

By employing the process of the present invention, the case program manager identifies patients receiving therapy and, more importantly, the subset of these who are not being treated in accordance with the preferred treatment regimen for the patient's disease state. This population of patients is very relevant to this invention; from this population, the treatment regimen of those patients who are not receiving the preferred therapy regimen can be automatically identified and influenced to change habits or conform to the recommended treatment regimen.

For convenience, in the subsequent description of the present invention, the case program manager is shown as a single source of external information such as that from the Modeling Guidelines 104 for the Predictive Health Outcome Modeling process 102, and for the Intervention and Medical Guidelines 105 for the process of Intervention of At-risk Patients 103.

Generally, most functions of the case program manager are automated and implemented by, for example, a dedicated computer system. However, end users may: provide external information as a disease management program is initiated, provide changed or new parameters to a disease management program based on experience, or modify intervention techniques as needed.

For most embodiments of this invention, the roles of the case program manager are divided among multiple persons or entities. For example, one "case management" entity can identify patients at risk for becoming "high-cost" patients, another entity can contact physicians with this information and with treatment advice as well as with patient educational materials and treatment compliance devices, and yet a third entity can contact physicians directly. Still another entity can be responsible for managing the identification of statistical information and creation of predictive models. As a result of carrying out the method of the invention, a larger number of patients receive appropriate therapies than would otherwise, and, consequently, a smaller number of patients suffer from serious disease progression requiring extraordinary, and expensive, care.

The method of the invention typically involves at least several treating physicians. One preferred embodiment includes approximately 100 treating physicians, but is also effective with larger numbers of physicians, e.g., 250 to 500 physicians, or more.

**The Disease Management System**

A high level flowchart of the Disease Management System of the present invention is shown in Figure 1B. Referring to Figure 1B, the Disease Management system includes a Disease Management Data Repository system 101 which includes the Patient Data Collection and Integration process 110 and the Disease Management Database 120. This is where the event-level information resides. Next, the Disease Management system includes a Predictive Modeling process 130, a Risk Stratification process 140, an Intervention Management process 160, and an Intervention Records and Tracking process 170.

A Case Management process 150 receives Intervention information from the Intervention Management process 160 and Results from the Intervention Recording and Tracking process 170. The Case Manager 150 provides externally derived information to the Predictive Modeling process 130 and Risk Stratification process 140.

The Disease Management Data Repository system 101 includes a Patient Data Collection and Integration process 110 and a Disease Management Database 120. The Patient Data Collection and Integration process 110 receives raw patient data from healthcare sources, and processes the raw patient data to remove redundant information and format the raw patient data into a common, predetermined format. Initially, one or more sources of information are required which allow for identification of an initial population of patients.

Typical sources of raw patient data may include, for example, healthcare providers such as doctors, hospitals, pharmacies, other healthcare providers, and payers who pay for these services which all keep records for their patients. These records, however, may be scattered, difficult to access, have different formats, and contain duplicate or incorrect information. Therefore, a more accessible source for such information exists in the health care claims records of a given benefits provider. These health care claims records are used in one exemplary embodiment of the invention.

The Patient Data Collection and Integration process 110 stores the formatted patient information in the Disease Management Database 120, which is the database storing the patient medical records, clinical data or other data used by the present invention.

The Predictive Modeling process 130 of the present invention uses an identified disease, statistical restrictions, and a sample patient database to create a predictive model and rules which can identify patients, from a predetermined identified disease patient population, who are at high risk to adverse health outcomes. As used herein, the term "identified disease" refers to a particular disease about which the client may be concerned, such as asthma, depression or congestive heart failure (CHF).

The Risk Stratification process 140 of Figure 1B applies a statistical predictive model and rules to patient data from the Disease Management database 120 corresponding to a group of patients selected from the Disease Management database 120 based on a predetermined criteria. The predetermined criteria could be "all client (MCO) patients" or "all new employees" for example. The Risk Stratification process 140 identifies a subgroup of at-risk patients and creates an intervention list from the subgroup.

The Intervention Management process 160 schedules and performs interventions for each identified patient on the intervention list, such as sending letters or educational materials, and making phone calls or home visits, to these at-risk patients. Finally, the Intervention Records and Tracking process 170 keeps a record of the interventions performed and their effects.

The operation of the Disease Management System as illustrated in Figure 1B is now described.

First, a particular disease of concern, as well as other predetermined restrictions, are identified by the Case Management process 150. The identified disease and restrictions are supplied to the Predictive Modeling process 130. The Predictive Modeling process 130 receives a subgroup of patient medical data from the Disease Management Database 120 corresponding to patients having the identified disease and meeting other predetermined statistical criteria determined from research data. The Predictive Modeling process 130 then creates a predictive model and rules from the subgroup of patient medical data which can identify patients from a predetermined identified disease patient population who are at-risk to adverse health outcomes.

The Risk Stratification process 140 receives the output predictive model and rules from the Predictive Modeling process 130, and further rules from the Case Management process 150. Based on the information provided by the Case Management process 150, medical and clinical information for a group of patients contained in the Disease Management database 120 is retrieved, and the group of patients is the predetermined client's identified disease patient population. The Risk Stratification process 140 then uses the predictive model and rules to identify a high-risk subgroup of patients from the predetermined client's identified disease patient population who are at-risk of adverse health outcomes.

Identifying a high-risk subgroup is a subjective undertaking which is defined by the operator. It is not a procrustean bed. For example, "high-risk" can be determined based on the severity of the disease or condition. Or it can be driven by available resources; there may be only so many resources available versus the cost of providing useful interventions. A classic example of "high-risk" is the triage approach used in dealing with major catastrophes: don't treat those who will die anyway, don't treat those who will live anyway; treat those for whom available intervention may result in survival or a lessening of permanent disability. Another example is to define the high-risk subgroup as comprising a certain percentage of the total group based on how many patients a particular operation can handle. So if the throughput of a particular system can only handle or manage interventions in 1000 patients on a given day, then the 1000 patients most in need out of the total population will be defined as the "high-risk" subgroup. In a similar way, the intervenor may have only enough money to usefully intervene in 1000 patients in six months. Hence by definition the 1000 patients most at risk become the "high-risk" subgroup. Another example is one where clinical outcomes are ranked from 1 to 5 in terms of possible useful outcome, and it is decided that those with a possibility of a good outcome ranking of 3 or greater should be progressed as the "high-risk" subgroup. In addition, age and age-related likelihood of an adverse outcome, or a positive outcome, may be used in defining a "high-risk" subgroup. For example it may be decided to define high-risk as those who are female, past menopause, and have a family history of an estrogen-dependent disease. And 2 or more of these factors will usually be combined in creating the algorithm for identifying the "high-risk" subgroup. These are but a few examples of how one might define "high-risk".

It should be noted also that although this step is described in terms of identifying a single "high-risk" subgroup, graded levels of intervention can also be defined and factored back into this analysis. So rather than defining a high-risk subgroup, one could define a set of subgroups where each was accorded a particular risk factor, and then intervention carried out on a selected set of subgroups based on different levels of accessed risk.

Once the high-risk subgroup, or a set of target subgroups, has been identified, the Risk Stratification process 140 creates an intervention list ranking the patients according to a predetermined criteria. The intervention list is used by the Intervention Management process 160 of the present invention to schedule and perform interventions, such as sending letters or educational materials, and making phone calls or home visits, to these high risk patients to prevent and/or improve their likely health outcomes.

The Intervention Management process 160 takes a data feed from the Disease Management Database 120, and the data is "client" identified disease patient data which is normalized into the Disease Management Data Repository format. This data feed or detection process has parameters and rules received from the Case Management process 150 that identify a specific patient meeting the conditions for participating in a disease program. This detection process provides a population for consideration in the specific identified disease program.

The Intervention Management process 160 also passes intervention contact data back to the main Disease Management Database 120 and the intervention list to the Case Management process 150. This intervention contact data is used in the analytic process to, for example, determine the success of the particular form of intervention.

The Intervention Record and Tracking system 170 keeps a record of the interventions and their effects, from which the Case Management process 150 can update external information used by the Predictive Modeling process 130, as well as guidelines for interventions, and the Best Practice Guidelines to improve treatment regimens for an identified disease.

The following sections describe in detail each of the processes of the Disease Management system of the present invention, as illustrated by Figure 1B.

**The Disease Management Data Repository and Data Integration**

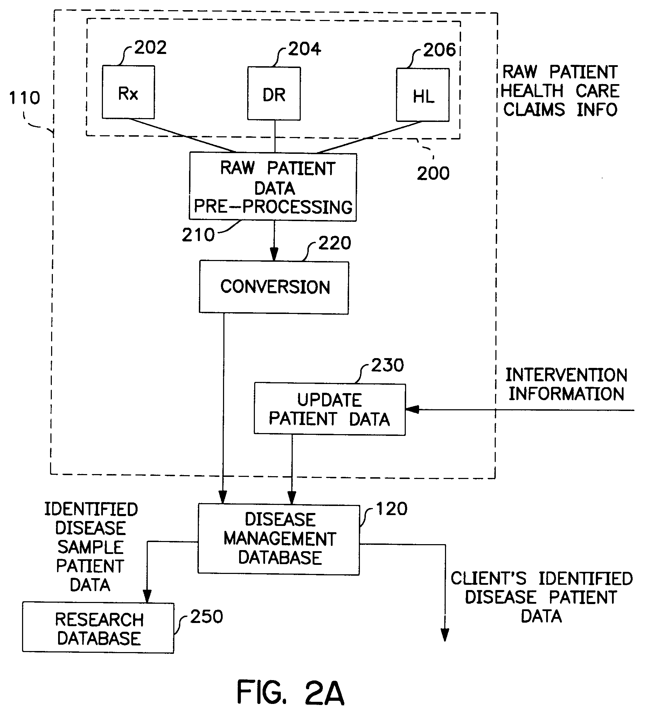

The Disease Management Data Repository 101 is described with reference to Figure 2A, which is a high level flowchart illustrating the raw patient data acquisition, pre-processing, and database formation of the present invention. The Disease Management Data Repository 101 includes the Patient Data Collection and Integration process 110, Disease Management Database 120, and a Research Database 250.

The Patient Data Collection and Integration process 110 includes Reimbursement Claims sources 200 as a data source, a raw Patient Data Pre-processing process 210 to "clean-up" the raw patient data, a Conversion process 220 for converting the raw data to a predetermined format, and an Update Patient Data process 230 to update patient information due to subsequent events or interventions (also called event-level information).

In the Patient Data Collection and Integration process 110, Reimbursement Claims sources 200 provide raw patient data to the raw patient data pre-processing algorithm. The exemplary sources of information, which allow for the identification of a population of patients who are currently provided medical treatment, are the clinical records and the health care claims records of many healthcare benefit providers. As is known, claims for drug reimbursement, doctor visits, hospital stays, and laboratory tests are received and processed for payment/reimbursement. In the exemplary embodiment of the present invention, this claims information is entered into, for example, a DB2 or Sybase database on a computer system (not shown).

The present invention is not limited, however, to these Reimbursement Claims sources 200 as shown. In another embodiment of the invention, data concerning individuals, such as demographic data; social data; personal data such as lifestyle, a history of sexual abuse or parental neglect or physical abuse, nutritional status; geographic data; family history; or other data can be used to populate the Disease Management Database.

The method of the invention is typically carried out with the assistance of an electronic database for storage, and retrieval, of data concerning an individual, such as medical data, demographic data, pharmaceutical data, diagnosis data and treatment data, from reimbursement claims sources 200. For example, the following pharmaceutical data can be retrieved from reimbursement claims:

    a) patient identifier
    b) drug prescribed
    c) drug dosage
    d) amount of drug
    e) duration of drug therapy
    f) dates of recent prescription fills/refills
    g) provider identifier.

The data are stored preferably in machine-readable form and are recoverable in discreet, searchable fields with a discreet record for each patient. Each record also preferably comprises a field for noting whether or not one or more case management interventions as described herein have been undertaken. The data are stored in a computer and accessed through customized database utilization software. Such software provides searching and reporting (display, printing, and electronic distribution) capability.

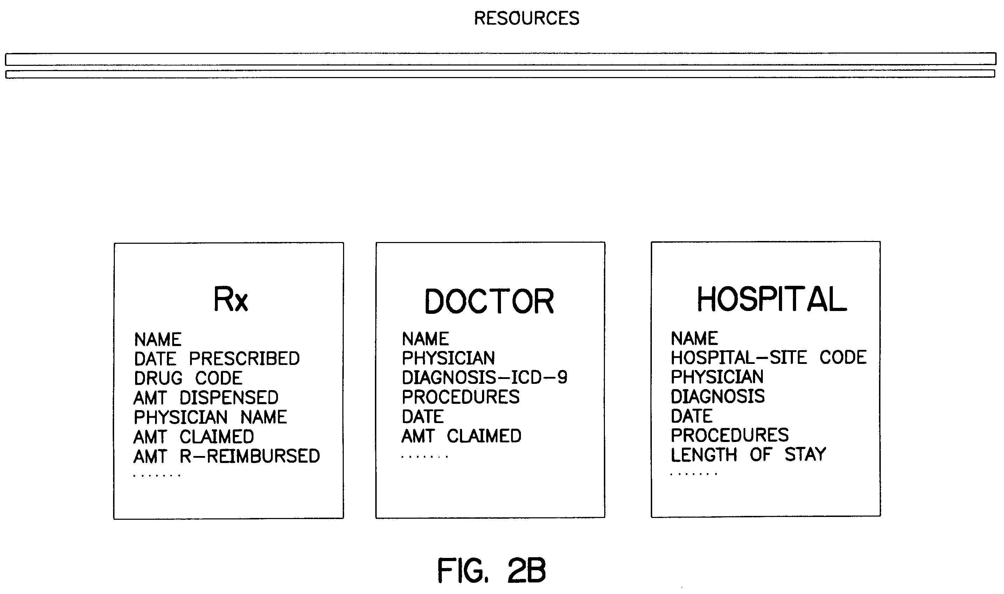

Figure 2B is a high-level block diagram illustrating three exemplary sources of information suitable for use with the present invention. As is illustrated in Figure 2B, the claims information of such a provider would typically include three sources: pharmacy (Rx) claims 202, doctor (DR) claims 204 and hospital (HL) claims 206. As listed on the blocks representing the claims information, many types of information would be available from the respective claims including drug codes, physician's names, diagnosis codes, procedures, various dates and other relevant information. Much of this information is referenced using codes, such as drug codes, procedure codes and illness codes.

Continuing with Figure 2A, the Raw Patient Data Pre-processor 210 performs data integrity checks which identify and process rejected or reconciled claims.

To make the use of the database more efficient, the database utilization subalgorithm (not shown) of the Raw Patient Data Pre-processing algorithm 210 has the capability of eliminating redundant entries, of eliminating entries for patients who have become ineligible and of ignoring records for which a case management intervention has been undertaken within a preset period of time.

Second, the Conversion algorithm 220 reads the source data files and populates the Disease Management Database 240 with the patient information in a predetermined database format. The Disease Management Database 240 of the present invention uses Sybase, but any similar database product may be used.

Finally, the Update Patient Data process 230 of Figure 2A receives intervention management information from the Intervention Management process 160 and Intervention Recording and Tracking process 170 and updates the patient information of the Disease Management Database 120 to include information about interventions regarding the member patient.

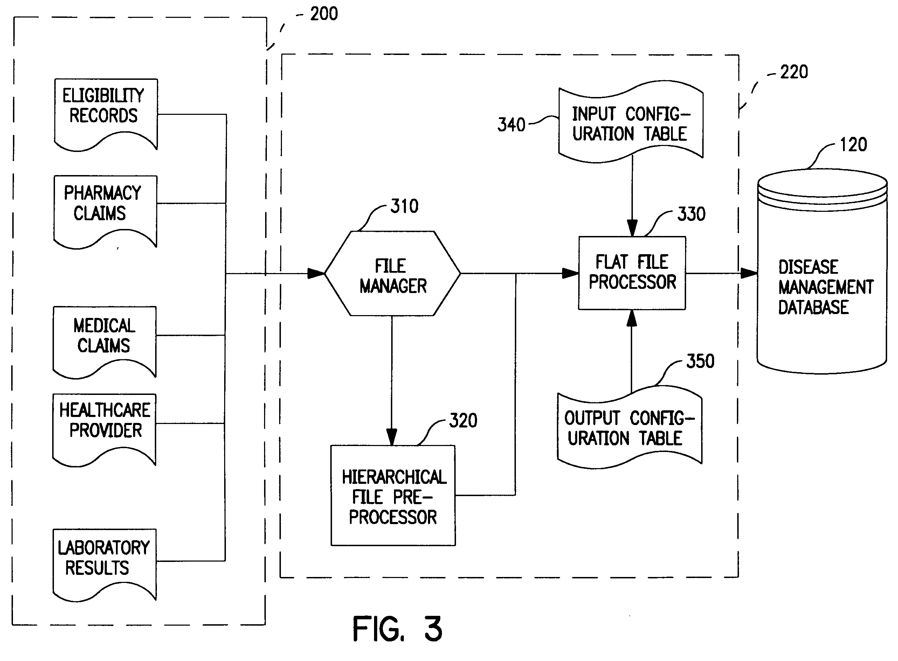

A more detailed flowchart of an exemplary embodiment of the Conversion process 220 is shown in Figure 3.

Referring to Figure 3, the File Manager 310 receives patient data files and identifies the incoming files, verifies that they are suitable for processing, and stores information about each file in a file inventory database. If the file is Hierarchical, the File Manager 310 sends the file to the Hierarchical File pre-processor to read the contents into flat files. The flat files are then stored into the Disease Management Database 240 by the Flat file Processor 330 using the information contained in the Input configuration table 340 and Output configuration table 350. The patient data is then stored in the database using a Data Model.

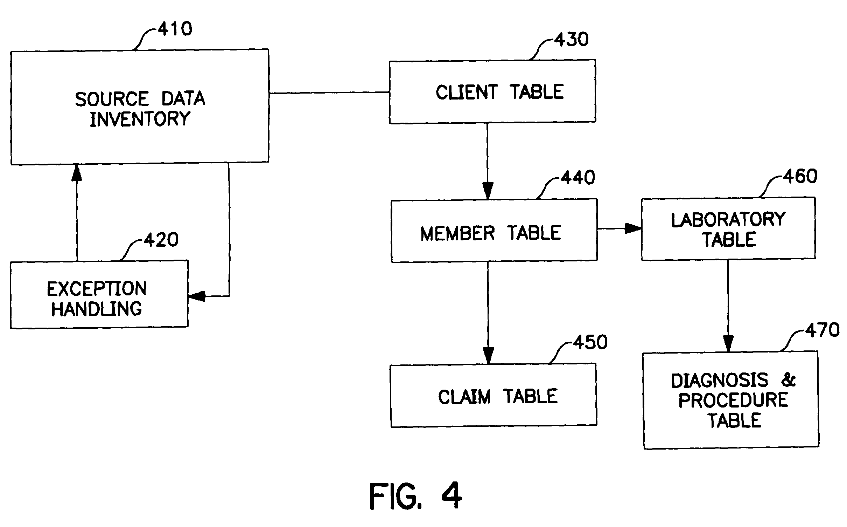

Figure 4 is an illustration of an exemplary Data Model as used in the patient data repository of an embodiment of the present invention. The Data Model includes a Source Data Inventory 410, which records aspects of incoming data during database population; an Exception Handling process 420 which handles data exceptions during the population process; Client Tables 430, which contain lists of the Disease Management provider clients; and a Member Table 440, which includes member specific identity information.

The Data Model also includes, for each member patient in Member Table 440, a Claim Table 450, which is a record of healthcare activity for a single member; a Laboratory Table 460, which represents the entities and relationships involved in gathering clinical test data for a given member; and a Diagnosis and Procedure Table 470, which contains a record of related diagnoses and medical procedures for a given claim.

The organization process of the Data Model is as follows. Referring to Figure 4, the source Data Inventory 410 records the progress and nature of incoming data during the database population process. The Exception Handling 420 handles data exceptions during the population process. The exception may be caused by missing values, values out of range, or other errors in the data, and the Exception Handling 420 resolves these exceptions when they occur by throwing away the data, retaining some of the data, or resolving the errors based on available information. The Source Data Inventory 410 provides received client data to populate the database with Client Tables 430.

The Client Tables 430 contain lists of the Disease Management provider clients which have patients and are subscribing to the system and method described in the invention. Each client in the Client Table 430 has patients defined as members in the Member Table 440. The Member Table 440 includes such information as member name, date of birth, and gender.

For each member patient in Member Table 440, a Claim Table 450 is kept. Each claim in Claim Table 450 is a record of healthcare activity for a single member. Data items recorded are, for example, dates when the claim was initiated or resolved, drug and prescription information, details of a medical examination, the member's primary or other physicians, and encounter services or procedures provided.

In addition, the Laboratory Table 460 represents the entities and relationships involved in the requisition, accession, and resolution of laboratory tests performed for a given member. Data items recorded are, for example, blood tests, glucose tests, or other tests based on a single analyte.

Finally, the Diagnosis and Procedure Table 470 records primary and one or more secondary diagnoses for a given claim, which are expressed as ICD-9-CM codes. Diagnoses can be grouped together into a Diagnosis-Related Group (DRG), and a DRG is one of 495 classifications of diagnoses in which patients demonstrate similar resource consumption and length of stay patterns. The Diagnosis and Procedure Table 470 also records procedures corresponding to each diagnosis, and these procedures can be expressed as out-patient CPT codes, in-hospital HCPCS, or other proprietary codes.

A second, identified disease specific database is created for the purposes of providing a database of identified disease patient data for the Predictive Modeling process 130. Returning to Figure 2A, this database is the Research Database 250 which is a claims level database in a predetermined format, such as SAS format. Although Figure 2A shows that the identified disease sample patient data used to populate the Research Database 250 is provided by the Disease Management Database 120, the present invention is not so restricted and the Research Database 250 can be populated from Reimbursement Claims sources 200 using an appropriate pre-processing algorithm.

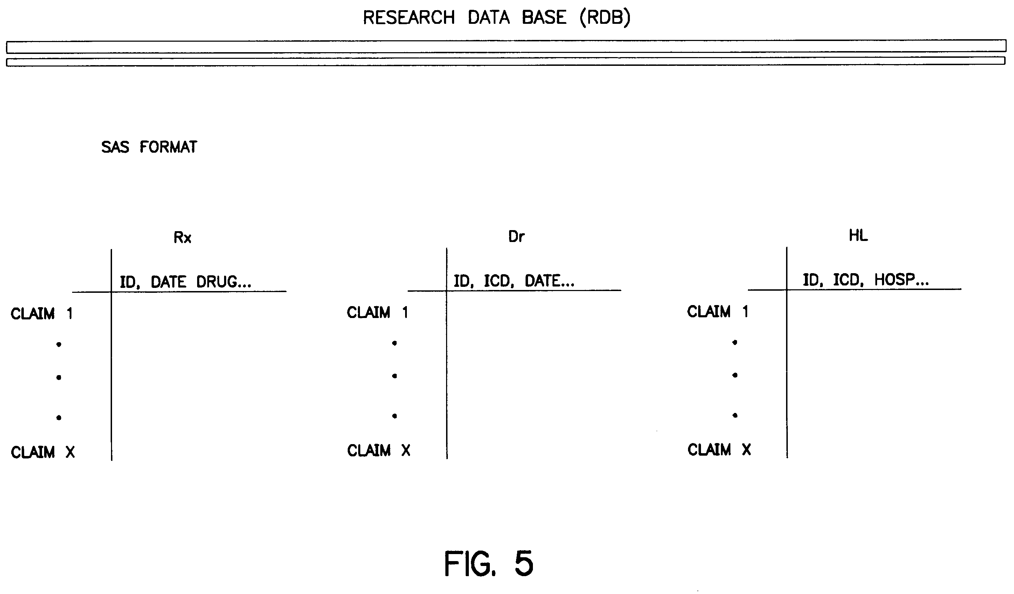

Exemplary formats illustrating the research database format for each of the Rx, DR, and HL claims of the records contained in the research database are shown in Figure 5. As shown in Figure 5, claims are listed from claims 1 to claim x and the appropriate information, for the particular service provider being claimed, is also presented. The DB2 database still represents a source of raw data elements which require processing by the raw patient data pre-processing algorithm 210. Subsequently, the data is routinely downloaded into a Research Database 250.

**Creation of Predictive Models**

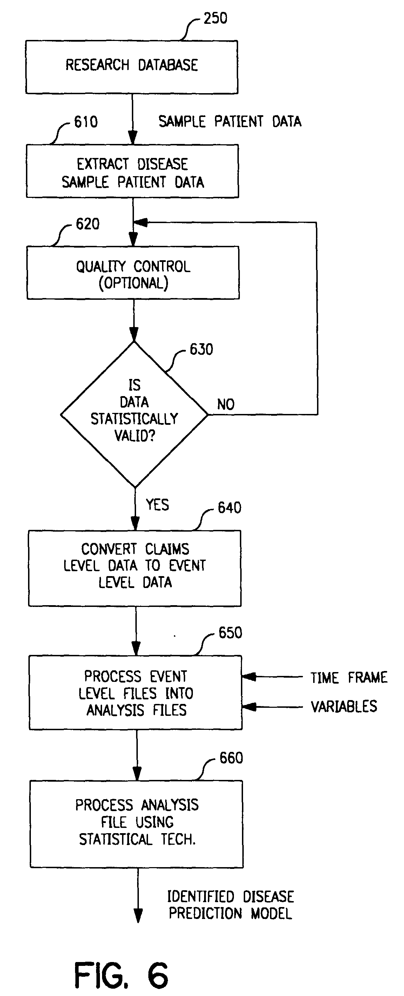

Turning to the statistical prediction modeling, Figure 6 is a high level flowchart illustrating the sample patient data extraction process and predictive modeling process for an identified disease according to the present invention. As shown in Figure 6, the Predictive Modeling process 130 includes the steps of 1) Extracting Identified Disease Sample Data 610; 2) Performing a Quality control operation (optional) 620; 3) Checking Whether the Data is Statistically Valid 630; 4) Converting Claims level data into Event Level Data 640; 5) Processing the Event Level Files into Analysis Files 650; and 6) Processing the Analysis File using Statistical Techniques to create an identified disease prediction model and rules.

Referring to Figure 6, the process of determining a predictive model begins with step 610, Extracting Identified Disease Sample Data. The extraction process of step 610 receives the sample patient data from the Research Database 250 and an identified disease from the Case Management process 150 when the data has been converted to SAS format, SAS procedures process the information to: 1) extract patients with the identified disease (step 610), 2) process the claims level information into event level information (step 640), 3) using predetermined variables and timeframe schemes, generate analysis files for analysis purposes (step 650) and 4) create a prediction model as a function of those variables most reflective of the correlation to an adverse health outcome (step 660).

It should be mentioned that, from a statistical perspective, an important consideration in developing prediction models from datasets is sample size. To maximize the integrity of the prediction model, a valid sample size is an important factor, and sample sizes required to determine prediction equations depend on the magnitude of association between variables. As these associations are unknown, all patients within any individual plan are initially included.

The first step, extracting patients with an identified disease or condition (step 610), uses various parameters provided either by the case program manager, research source, or other healthcare professional to define which patients qualify for the overall initial universe of patients with the identified disease to be considered.

For example, in one exemplary embodiment of the present invention, only patients having a continuous enrollment with the benefits provider of 12 months or longer and having a claim for depression or treatment with anti-depressant medication are eligible. Of course, these criteria are exemplary and could be modified such that 24 months or 6 months of enrollment is satisfactory or that an individual must be 18 years of age. In the exemplary embodiment of the present invention, the Extracting of Identified Disease Sample Data, step 610, extracts all claims data for patients with either an appropriate code for an identified disease (such as depression; see Appendix I) or for treatment with a drug used in treatment of the identified disease (for example, for depression, an antidepressant drug; see Appendix III).

It should be noted that in the health care industry various codes are used in claims information for indicating which procedures, treatments, diagnoses, drugs, etc. are being claimed. For the exemplary embodiments of the present invention, examples of the selected codes are shown in Appendices I and II. These codes were found in Physician's Current Procedural Terminology (CPT), American Medical Association (1995) and St. Anthony's ICD-9-CM Code Book (1994) which are both hereby incorporated by reference for their teaching of codes and sources of codes. As will be appreciated by those skilled in the art, any set of codes, representative of the various procedures, treatments, diagnosis, drugs, etc. relevant for use with the present invention would suffice. References to such codes occur throughout this specification.

Subsequent to the extraction process of step 610 of Figure 6, the claim adjustment and integrity checks are optionally performed in the data Quality Control step 620. The Quality Control step 620 is optional, as, for example, the patient data for an identified disease may not require the step or the original Disease Management Database 120 may already be of sufficient quality due to the raw Patient Data Pre-processing step 210 (shown in Figure 2).

One method of Quality Control of step 620 generates, from the dataset defined above, intermediate output files which contain sets of frequency counts for processing purposes. In one exemplary embodiment of the present invention, with depression as the identified disease, intermediate output files for the following characteristics are generated for review:

a. frequency counts of unique members by sex, age groups (0-9, 10-19...) and enrollment duration by months including:

i) Tables showing count of members by sex, ii) Table showing count of members within age groups, iii) Table of counts of age groups broken down by sex, iv) Table of enrollment duration by months i.e., 1 month to maximum number of months possible.

b. frequency counts of ICD codes for depression (Appendix I), i.e., number of members having at least one hit with each of the ICD codes in Appendix I-a any level ii) as first code.
c. frequency counts of anti-depressant drugs (Appendix II):

i) number of members who have at least one claim for each of the drugs in Appendix III.

d. count of members who became eligible for processing due to ICD code only, by drug only, and by both ICD code and drug.
e. frequency counts of numbers of all claims within each file (HL, DR, Rx) by member.
f. frequency counts of ICD codes (use only the first 3 digits of ICD codes) of any nature in DR (any position) and HL files - at least the top 10 with frequency of each. i.e., 2 tables one each for DR and HL files.
g. frequency counts of hospitalizations by calendar month. Counting calendar month backward from last month of eligibility or data availability. The last month for which data is available will be month 1, the penultimate month with be month 2 etc.
h. frequency counts of procedures related to depression (CPT codes, Appendix I-b).
i. frequency counts of all CPT codes (to the level of the first 3 code digits) - at least the top 10.

The above frequency counts for use in performing preliminary evaluations as to the integrity of the data are exemplary and could be modified to include/exclude parameters which are shown to be more/less useful.
In another exemplary embodiment of the invention, with Congestive Heart Failure as the identified disease, the following frequency counts are generated:

A) First, a frequency count of the number of enrollment periods for the members is generated. Then, for members with multiple enrollment periods of at least 6 months duration, it is determined if a CHF diagnosis is present in each enrollment period. Consequently, enrollment periods without a CHF diagnosis are excluded and, for members with multiple enrollment periods that have a CHF diagnosis, only the most recent enrollment period that contains a CHF diagnosis is kept.
B) For the one enrollment period for all remaining members, all costs, denoted ALL COSTS, encountered by that member during the entire enrollment are identified. A complete proc univariate for ALL COSTS is provided for each plan separately and all plans together. It should be noted that "proc univariate" is a SAS procedure which generates descriptive statistics (e.g., mean, standard deviation, etc.)
C) From the ALL COSTS determined above, costs which are specifically cardiovascular (CV), denoted CV COSTS, are identified. In doing so, a cost is considered to be a CV COST if a claim from the DR or HL file has any CV ICD-9 code in the first or second position. If a claim from the Rx file is from therapeutic class 04000 then it is counted as a CV claim and count cost as a CV cost. A complete proc univariate for CV COSTS is also provided for each plan separately and all plans together.
D) From the CV COSTS, those costs which are specifically congestive heart failure related, denoted CHF COSTS are identified. A cost is considered to be CHF COST if a claim from the DR or HL file has any CHF ICD-9 code in the first or second position. A complete proc univariate for CHF COST is also provided for each plan separately and all plans together.
E) For all member enrollment periods remaining, the total member months for each plan is calculated separately and together. In doing so, a member is considered enrolled during any month that they were enrolled for at least one day. For this, a complete proc univariate is provided for member months for each plan separately and all plans together.
F) Finally, a unique member count is provided for all patient status code = 20 within the remaining enrollment periods. It is noted that status code = 20 indicates that the patient has expired or did not recover.

It should be noted that, regarding the cost calculations, the following guidelines apply in the exemplary embodiment of the present invention:

a. the cost of inpatient hospitalizations, emergency services, physician/outpatient, and other medical services on a per claim basis are considered to be:
        AMTPAID + AMTCOPAY + AMTRESERVE + AMTDEDUCT
b. the cost of drugs are considered to be:

AMTPAID + AMTCOPAY

where AMTPAID is the amount paid, AMTCOPAY is the amount co-payed, AMTRESERVE is the amount reserved and the AMTDEDUCT is the deductable amount.

It should also be noted that, for purposes of a cost hierarchy, the following rules were used in the exemplary embodiment of the present invention.

1. Only hospitalizations for CHF can spawn other events.
2. Hospital costs include all Rx, procedure, physician charges.
3. Hospital visits can generate Rx and procedure events with costs set to zero (included in hospital cost).
4. Hospital visits cannot generate separate doctor visit events.

Once again, the above information, which is used to perform preliminary evaluations as to the integrity of the data, is exemplary and could be modified to include/exclude parameters which are shown to be more/less useful within the spirit of the present invention.

With this information, a "quality check" is performed on the initial universe of identified disease patients to make sure that the final results, i.e., prediction model, is not unreasonably skewed due to invalid input information. This processing for maintaining data quality, Quality Control step 620, produces intermediate output files, and allows for a refinement of the extracted information by, for example, checking to see if an imbalance exists in the extracted information such as all claims are from individuals over 60 years of age, all claims are from men, or other data imbalances which would otherwise taint the integrity of a prediction model. Step 620, in the exemplary embodiments, is performed manually by viewing the intermediate output files. It is contemplated, however, that using various threshold values, the frequency counts can be automatically scanned for a potential imbalance.

Having now extracted and refined the claims level information according to various predetermined criteria deemed relevant for subsequent processing purposes, the information is converted into an event level format.

Returning to Figure 6, the next step is the Convert Claims Level Data to Event Level step 640. To provide processing flexibility, particularly in assigning time windows for analysis, the above-mentioned second step (i.e., converting the claims level information into event level information, step 640) is employed to generate two primary data files from which an analysis file can be created.

In the exemplary embodiment of the present invention, primary data file 1 is a member level file and contains all data of a static nature (i.e., not time sensitive) such as 1) Member Key, 2) Date of birth, 3) Gender, 4) First available date of enrollment (i.e., start of dataset (1/1/92) or enrollment date), 5) End date of enrollment (i.e., end of dataset or last date of enrollment), 6) Date of first identified disease event (for example, first prescription for antidepressant, or hospitalization for congestive heart failure), 7) Date of last hospitalization, 8) Number of records in events file (primary file 2), and 9) Mode of entry into the dataset (e.g., i) Anti-depressant drug only, ii) Depression diagnosis only, iii) Both anti-depressant drug and depression diagnosis).

Primary data file 2 is an event level file with a record for each event ordered by member and the chronological date of the event, and, in the present invention, presented in descending order of event date.

It should be noted that an event, sometimes referred to as an episode, is an occurrence which, based on clinical knowledge, is deemed relevant to the identified disease. Having knowledge of what raw data elements are available from the claims, a set of events is defined directly or indirectly from the data elements where events can be based on an individual data element, a combination of data elements or it can be derived from individual or multiple data elements.

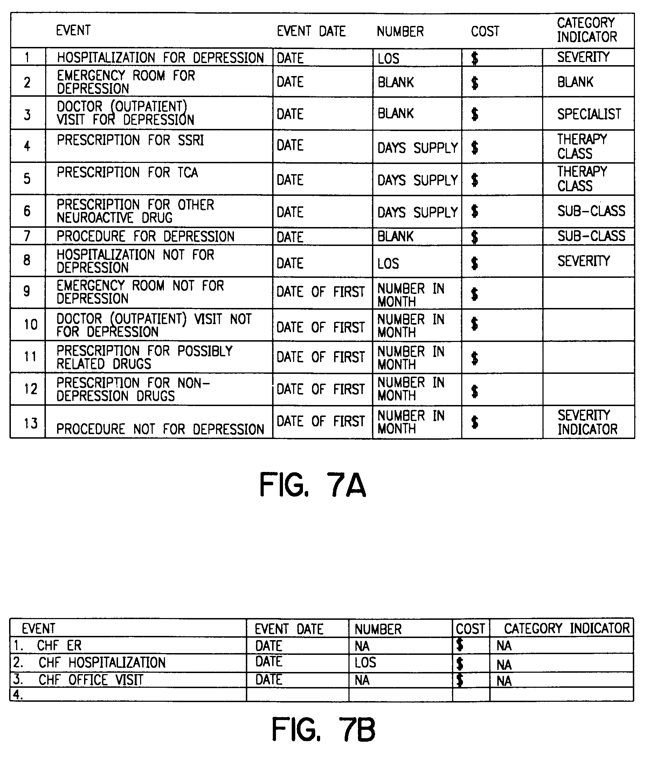

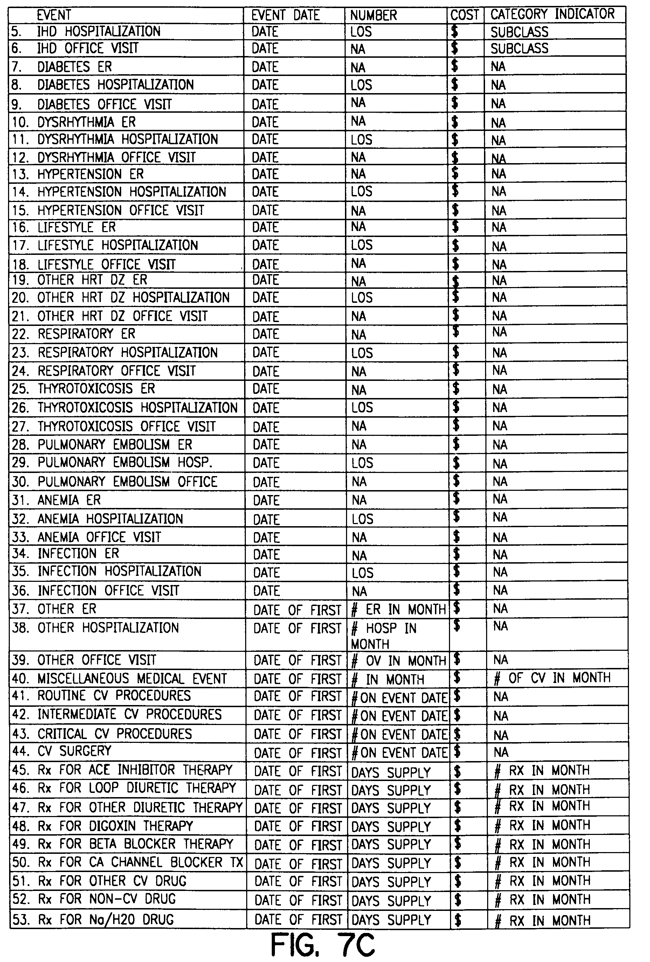

Figure 7A is an exemplary list of events and format for primary file 2 (an event level file) for depression as the identified disease. As shown in Figure 7A, the entries provided include:

1. Hospitalization for depression

a. Any hospital claim identified by hospital site code.
b. Having a from and through duration of at least 1 day.
c. Having ICD 9 code.
d. Depression ICD 9 code occurring at any position.
e. Illness indicator (Appendix V) 1 = major illness, 2 = suicide, 3 = major illness and suicide. 0 = everything else.

2. Emergency room for depression

a. Emergency room visit identified by emergency room site code.
b. Having ICD 9 code (see Appendix I-a).

3. Doctor (non-hospital) visit for depression

    a. Any doctor claim.
    b. Having ICD 9 code (see Appendix I-a).
    c. Category: Psychiatrist =- 1, all others = 0.

4. Prescription for SSRI

    a. SSRI (selective serotonin re-uptake inhibitors) therapeutic class 5.51.3.
    b. Cost = 0 if generated from a hospital admission.
    c. Category indicator = blank

5. Prescription for (Tricyclic antidepressants) TCA or (Monoamine Oxidase inhibitors ) MAOI

    a. Therapeutic classes 5.5.1.1 (tertiary amines), 5.5.1.2 (secondary amines), 5.5.1.4 (Monoamine Oxidase inhibitors). AND 5.5.2
    b. Cost = 0 if generated by a hospital admission
    c. Category indicator therapeutic class 1 = 5.5.1.1, 2 = 5.5.1.2, 3 = 5.5.1.4, 4 = 5.5.2

6. Prescription for other neuroactive drug (From Rx file)
7. Procedure for depression (from DR or HL files)

| Category: | CPT codes or ICD procedure |
|---|---|
| 0 = Psychotherapy | All CPT and ICD codes in Appendix I-b not listed below. |
| 1 = Diagnostic | 90801, 90820, 90825, 90830, |
| | 90862 |
| | 94.0x, 94.1x, 94.21, 99.22, |
| | 94.23 |
| 2 = Shock therapy | 890870, 908712 |
| | 94.24, 94.26, 94.27 |

    For this entry, costs are assigned to the doctor visit or hospitalization in which the procedure occurred.
8. Hospitalization not for depression
    It should be noted that items under entry 8 could have been performed for a condition other than depression although these patients got into the cohort by virtue of receiving a depression diagnosis or receiving and antidepressant at some time making it likely these procedures were for depression.

    a. All hospitalization having from and through dates of at least one day duration.
    b. Major illness ICD 9 codes (see Appendix V).
    c. Category as in 1 above (1 = major, 2 = suicide, 3 = both, 0 = all others)

    Counts for entries 9-13 are aggregated for each month. The date is that for the first occurrence of the identified events. In the number field, the number of identified events occurring in that month are summed.
9. Emergency room not for depression

    a. Emergency room visit identified by Emergency room

10. Doctor (outpatient) visit not for depression

    a. Any doctor visit.
    b. Excluding visit with a depression diagnosis (Appendix I-a) i.e., not in 3/above.

11. Prescription for possibly related drugs
    Drugs identified in Appendix IV
12. Prescription for all other (non-depression) drugs
    All drugs not included in Appendices III or IV.
13. Procedure not for depression (from Dr and HL files)

a. Category indicator 1 = major procedures, 2 = minor procedure (see Appendix IV).

Figure 7B illustrates the exemplary list of events and format for primary file 2 (an event level file) for the exemplary embodiment with congestive heart failure as the identified disease. This embodiment exemplifies that the primary files 1 and 2 can be subdivided using the following exemplary ground rules which provide counts for the various events:

I. Count as a hospitalization event, denoted HOSPITALIZATION, (using both 1st and 2nd ICD-9 codes) a claim having a from and through date of at least one day and having a site code of 04. It is noted that a site code distinguishes between the sites at which the service under consideration took place (e.g., emergency room, doctor's office, etc.). It should be noted that costs go to 1st ICD-9 code category only. Also, if a new hospitalization occurs within one day of discharge from a previous hospitalization, the two hospitalizations are bridged into one. If a new hospitalization occurs greater than one day following a previous hospitalization, the second hospitalization is considered a new one.

II. Count as an emergency room visit event, denoted ER VISIT, (using both 1st and 2nd ICD-9 codes) a claim having a site code of 07, 08 or 10 OR a claim with the following the Hospital Common Procedure Coding System (HCPCS) codes: A0010-A0070, A0215-A0225, A0999 with a provider code = 81. It should be noted that costs go to 1st ICD-9 code category only.

III. Count as an office visit event, denoted OFFICE VISIT, (using only one ICD-9 code) a claim having a site code of 01 or 06 and having a unique date of service (DOS) but allow for different provider keys on the same DOS (if same provider key on same DOS, consider to be the same office visit) BUT if an office visit event occurs during a hospitalization, do not generate an office visit event (Attribute all costs for this event to the hospitalization). ALSO count as an OFFICE VISIT a claim with the following HCPCS codes: A0080-A0210 with provider code = 81. For all other office visit events, costs go to 1st ICD-9 code category only. It should be noted that the following provider keys are not considered as separate office visits and should be bridged with an office visit that occurs on the same DOS if one exists: 1) 24 (therapeutic radiology), 2) 34, 35 (independent lab), 3) 55 (hosp o/pat lab x-ray).

The three event types illustrated above are then further defined according to the associated Diagnoses.

The next step of Figure 6 is the Processing of Event Level Files into Analysis Files, step 650. After generating the two primary files using the above described instructions corresponding to step 640, further processing using timeframe information and selected variables (independent and dependent variables) is performed on the event level data to generate an analysis file, at step 650.

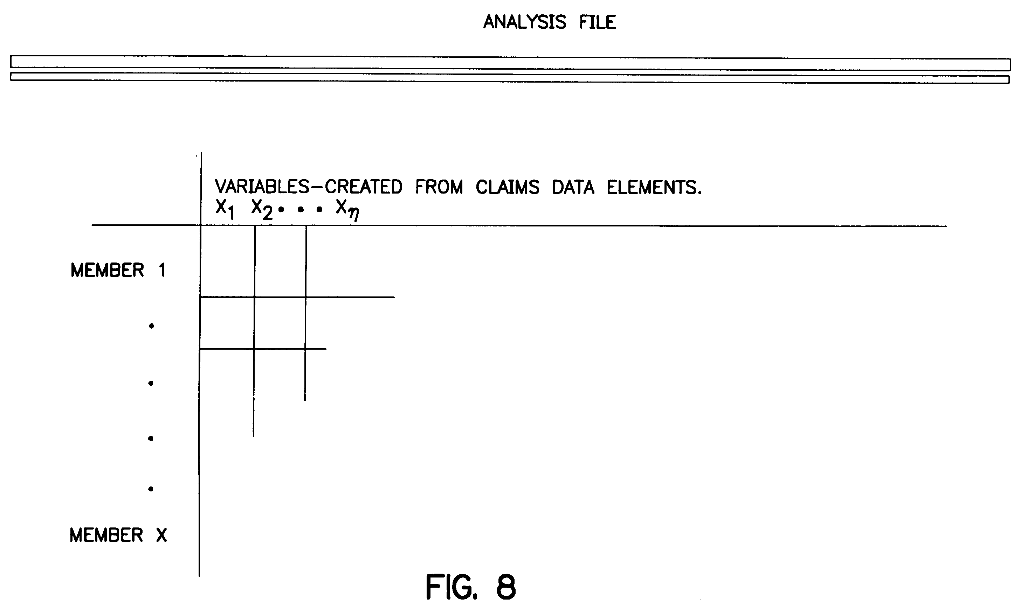

Figure 8 shows an exemplary format for the analysis file. As shown, the format of the analysis file includes a list of members in a first column of a table. Across the top of the table is a list of variables, described in detail below. The body of the table provides indications as to a member's relation to a listed variable.

In particular, the processing from the primary files to the analysis files in step 660 includes an algorithm defined, in part, by a time window and a plurality of variables. The algorithm can be re-programmed for various time window adjustments as well as variable modifications. The analysis file generated at this step is a member level file (i.e., organized with respect to members). The main analysis files are member level files derived from the information in the primary files.

Each main analysis file is created to take into account a single reference time window of censored events and prediction window of interest for that file. Each new time window applied to the data, in the exemplary embodiment, requires another main analysis file.

To generate the analysis file, a time window scheme, along with a plurality of variables, is applied to the event level data.

Discussing the variables first, included in the processing are both independent and dependent variables. The independent variables basically represent potential predictors of the adverse health outcomes; whereas, the dependent variables basically represent the adverse health outcome to be predicted.

To determine exemplary independent variables for step 650, as many of the original data elements as possible are used, assuming nothing about the identified disease. Then, based on clinical knowledge about the identified disease, additional variables are created. Furthermore, combinations of the data elements and/or variables, based on clinical knowledge, are used as variables. Finally, some variables may be created and used based on their potential utility as a leverage point in disease management.

In the exemplary embodiment of the present invention, the plurality of variables, in addition to each of the items in the event file, currently used by step 650 in the SAS routine for generating an analysis file for the exemplary embodiment with Congestive Heart Failure (CHF) as the identified disease are shown below in Table 1. It is noted that each of the events in Figure 7B is automatically considered an independent variable for processing.

Table 1

| Additional Independent Variables of Interest: | |
|---|---|
| 1. | Age (at time of 1st CHF diagnosis or drug therapy - one of the triple) |
| 2. | Gender (M/F) |
| 3. | HMO Membership (identification of particular HMO) |
| 4. | Site of first CHF diagnosis (site code) |
| 5. | Ischemic Heart disease (Y/N) |
| 6. | Diabetes (Y/N) |
| 7. | Adverse Lifestyle Diagnoses (Y/N) |
| 8. | Cardiac Dysrhythmias (Y/N) |
| 9. | Other Heart Disease (Y/N) |
| 10. | Hypertensive Disease (Y/N) |
| 11. | Number of Co-Morbid diseases (0-x) |
| 12. | Number of ACE inhibitor prescriptions (0-x) |
| 13. | Number of digoxin prescriptions (0-x) |
| 14. | Number of loop diuretic prescriptions (0-x) |
| 15. | Number of other CV prescriptions (0-x) |
| 16. | Number of non-V prescriptions (0-x) |
| 17. | Medication Possession Ratio (Compliance measure) |
| 18. | Number of CHF hospitalizations |
| 19. | Number of CHF emergency services |
| 20. | Number of physician office visits |
| 21. | Total Costs |
| | In-Patient Hospital Costs |
| | Emergency Room Costs |
| | Doctor Costs |
| | Pharmacy Costs |
| 22. | Cardiovascular Costs |
| | In-Patient Hospital Costs |
| | Emergency Room Costs |
| | Doctor Costs |
| | Pharmacy Costs |
| 23. | CHF Costs |
| | In-Patient Hospital Costs |
| | Emergency Room Costs |
| | Doctor Costs |

Turning to the dependent variables, potential dependent variables, for example, contemplated for use with the present invention are results to be predicted. For CHF, such predicted results include:

1. Hospitalization (HL) for CHF. This is a dichotomous variable which is referred to as the HL indicator such that HL = 1 if an admission occurred, otherwise the indicator equals 0.

2. High Cost. For example, the High Cost indicator may be defined as the highest 10% of resource utilization measured in dollars. Resources counted from time of cost in the top 10% of the first CHF diagnosis or receipt of first CHF-related drug (in the record) + 1, 3 and 6 months - separate analyses for each time period. Again, this is a dichotomous variable referred to as the High Cost indicator such that if the patient, for example, is in the top 10%, High Cost = 1, otherwise High Cost = 0.

The High Cost indicator, in the exemplary embodiment, could also be defined as the distribution of total cost per member (PMPM) in the prediction region (B to C). The High Cost indicator is set to 1 for the 10% of members with the highest PMPM in the Total Cost distribution and set to 0 for all others.

3. Death.

Although only three dependent variables for the given example are listed above, as those of ordinary skill in the art will appreciate, other known or yet unknown variables consistent with the goals of the present invention may also suitably serve as a dependent variable within the scope of the present invention.

Turning to the time window aspect of the generation of the analysis file, it should be noted that there is one analysis record for each selected member.

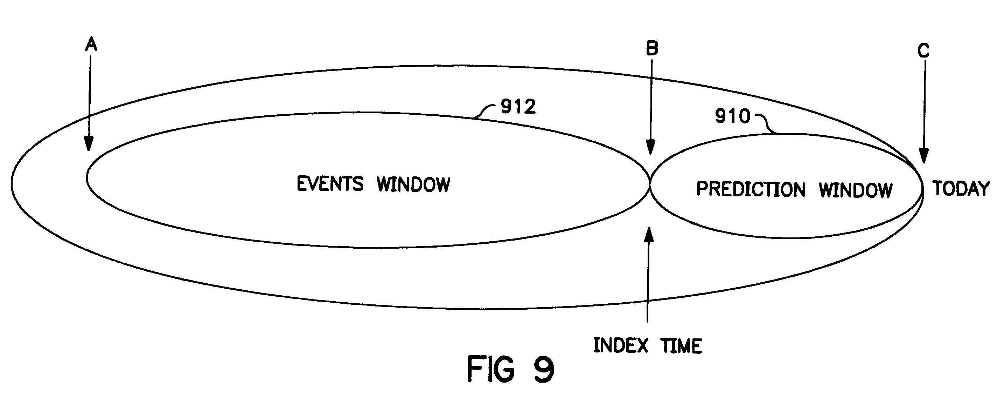

In the present invention, a scheme, as described below, has been developed for defining prediction zones and censoring data to create the analysis file. That is, referring to Figure 9, a time window basically defines a prediction zone or region 910 and an events window (analysis region) 912 from where activity is used to predict something in the prediction zone. As those skilled in the art will appreciate, additional time window schemes may also adequately serve the present invention.

For purposes of explanation, the time that the claims history covers is referred to as the time window that starts at some point 'A' and ends at point 'C'. The time interval is divided into analysis and prediction regions by point 'B' such that A<B≤C. That is to say, 'B' represents the present. 'A' represents the farthest past event and 'C' represents the farthest future event.

By way of example, Jane Doe's analysis record is based on claims from 1/1/91 through 6/30/93. Therefore, A=1/1/91, C=6/30/93 and B can be selected somewhere in between, such as 12/31/92. Generally, A is defined based on the data extraction protocol (i.e., from when the data is available) and C is defined by the last day for which the member is still enrolled and eligible for the benefits. Of course, variations of those general points of definition could be selected within the scope of the present invention.

The definition of the present instant B is important. In the subject invention, two basic definitions of B were devised in order to maximize the accuracy of the prediction model. Although, as would be understood by those skilled in the art, alternative definitions of B may also be used.

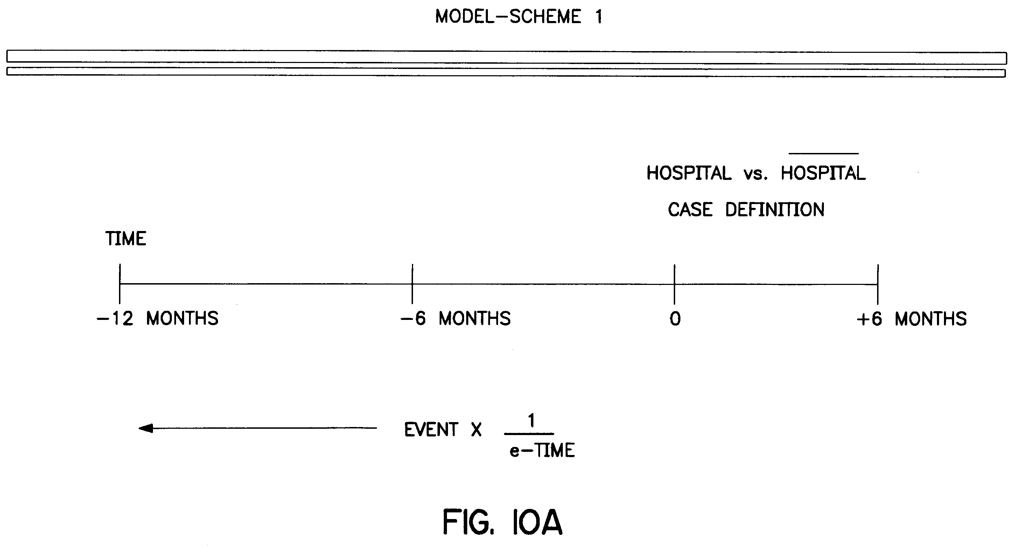

Figure 10A illustrates an exemplary time window scheme, referred to as Scheme 1, for use in processing the data from the event level files shown in Figure 6.

In Scheme 1, the event prediction region is set from B to C such that B=C-(x# of months) for all the members in the analysis. For example, if a 6-month CHF hospitalization (HL) model (i.e. HL is used as a dependent variable) is to be built then B=C-(6 months). In Jane Doe's example, B would equal 12/31/92. Therefore, only data covering from A through B (1/1/91-12/31/92) is used to predict the CHF in the 'next 6 months'. The phrase 'next 6 months' in this context implies that the time point B is "NOW" and any time after it is in the FUTURE and any time before it is in the PAST. This is a key concept of Scheme 1 and is important to understanding the prediction model implementation and application.

In alternative embodiments, analysis weights which reflect proximity to the event to be predicted can be used, for example. within 3 months x 1, 3-6 months x .75, 6-9 months x. .5,9-12 months x .25, greater than 12 months x .125. Other suitable weighting techniques, as will be appreciated by those skilled in the art, such as negative weights could also be used. For example, in the exemplary embodiment of the present invention, the actual weighting factor used is $1/e^{-x}$ where x = time in months from point B for each event.

Therefore, given a selected time window scheme and an appropriate set of predetermined variables, the processing step of 650 generates the analysis file.

Returning to Figure 6, once the Analysis files are generated in step 650, the next step, step 660, is to Process Analysis File Using Statistical Data, step 660, which provides the Identified Disease Prediction Model.

Using the analysis file, the model for identification/prediction can then be developed in various ways using statistical techniques. In particular, the analysis file, now at a member level, is processed using statistical functions available in SAS. In the exemplary embodiment of the present invention, the statistical processing performed to generate the prediction model is multiple logistic regression. As will be appreciated by those skilled in the art, other statistical techniques may also be suitable for use with the present invention.

In the exemplary embodiment, the statistical processing, when applied to the analysis file, identifies variables which meet predetermined levels of significance (e.g., probability value < 0.05). These variables then form a prediction model which is a mathematical equation of the following form:

$$\text{Logit}(p) = a + bx_1 + cx_2 \ldots + zx_i$$

where x1...xi are the identified variables and a...z are there parameter estimates. An individual's probability (p) for the outcome under consideration is then determined using the following formula:

$$p = e^{-\text{logit}(p)}/(1+e^{-\text{logit}(p)}).$$

Using the above steps, several experiments were conducted. In one experiment, the results for a model based on Scheme 1 with all commercial members and using the HL indicator as a dependent variable were determined. The resulting independent variables, most likely to predict an adverse CHF health outcome, were 1) hospitalization for CHF, 2) loop diuretics - days supply, 3) hospitalization for hypertension -length of stay, 4) doctor visits for CHF, 5) doctor visits for MI, and 6) ACE inhibitor possession (negative indicator).

In another experiment, the results for a model based on Scheme 1 with all commercial members with no prior CHF hospitalization and using the HL indicator as a dependent variable were determined. The resulting independent variables, most likely to predict an adverse health outcome, were 1) loop diuretics - days supply, 2) doctor visit for CHF, 3) hospitalization for IHD, 4) doctor visit for IHD, 5) emergency room visit for diabetes, 6) hospitalization for hypertension - length of stay, 7) emergency room visit for lifestyle, 8) hospitalization for other heart diseases, 9) doctor visit for pulmonary conditions, 10) doctor visit for anemia/emergency room visit for anemia, and 11) prescription (Rx) for "other" CV drugs.

In still another experiment, the results for a model based on Scheme 1 with Medicaid members and using the HL indicator as a dependent variable were determined. The resulting independent variables, most likely to predict an adverse health outcome, were 1) hospitalization for CHF, 2) loop diuretics - days supply, 3) doctor visits for CHF, and 4) emergency room visit for diabetes.

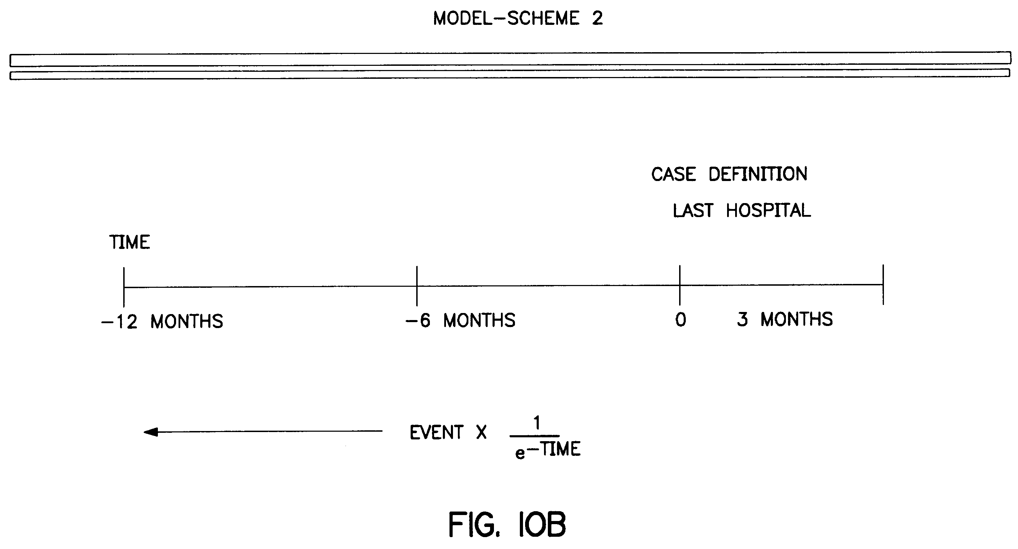

An alternative to Scheme 1, and referred to as Scheme 2, is illustrated in Figure 10B which shows a second exemplary time window scheme for use in processing the data from the event level files generated in the present invention.

A difference between Scheme 1 and Scheme 2 is the definition of the prediction region for members which have at least one identified disease hospitalization or emergency room visit (HL/ER). The prediction region starting at point B, in Scheme 2, is defined in multiple passes over each member's record. Turning again to Jane Doe's analysis record (from 1/1/91 through 6/30/93, A=1/1/91, C=6/30/93) to illustrate how this aspect works for defining point B, assume that Jane Doe was hospitalized for depression three times: on 4/1/91, 4/1/92, and 4/1/93.

Point B is set equal to the date of the first identified disease HL/ER - 1 month or set equal to point C if a member never had the identified disease HL/ER in their claims history. For Jane Doe, B=4/1/91. In the exemplary embodiment of the present invention, moving back one month from the HL date is performed to simulate the model application environment. There would probably be at least 30-day lag from model scoring to the disease management actions based on the scoring reports. Thus, in Jane Doe's record B=4/1/91-(1 month)=2/28/91. Jane's record, in this case, would not be used in the model building because the time span of the analysis region is only two months--less than the exemplary six month data history requirement.

Repeating steps 1 and 2 using second (or third or...) HL date to set point B, Jane Doe's record would eventually make it into model building on the second and third pass. This process, in the exemplary embodiment, terminates after three or four passes since there would probably be very few members with five or more identified disease HL/ERs in the study population.

It should be noted that the consequence of repeated modeling introduces added complexity of setting up additional independent variables. An important advantage, however, of Scheme 2 is that the prediction HL/ER rate would likely be higher than in Scheme 1.

In still another alternative embodiment, analysis weights which reflect proximity to the event to be predicted can be used, for example, within 3 months x 1, 3-6 months x .75, 6-9 months x. .5, 9-12 months x .25, greater than 12 months x .125. Other suitable weighting techniques, as will be appreciated by those skilled in the art, could be used. These type of weighting techniques may be used with either Scheme 1 or Scheme 2.

It should be noted that each of the experimental results indicate a different number of independent variables are used for the specific prediction models; and, depending on the precision of the models desired, more or fewer independent variables may be used based on their individual ability to accurately predict the selected dependent variable.

## Risk Stratification and Generation of Intervention Lists

Next, the determined prediction model is applied to the client specified data. The determined model can be applied to the existing data, to the data as it is regularly updated or to other claims databases for other benefits providers. To do so, only the determined independent variables of interest need to be processed. Of course, as new claims databases are to be analyzed, the entire process can be repeated to generate a new model in order to determine if other variables may be better predictors.

The output generated by applying the model is a file containing a list of all of the patients having the identified disease ordered by an indicator representative of the likelihood that that patient will have an adverse health outcome (i.e., experience that is defined by the dependent variable). This list can then be divided, for example, into subgroups such as in 5% or 10% increments of patients likely to have the adverse health outcome.

Model performance can now be assessed by determining the number of actual adverse health outcomes occurring in the prediction window for each 5% or 10% subgroup.

Applying the model to future claims data or other databases of identified disease patients or building a new model in a new database as described above, patients with an identified disease at high risk can be identified allowing for various types of intervention to maximize the effective allocation of health care resources for these patients. The Risk Stratification (RS) process 140 is required to generate such lists of patients, and the Intervention Management process 160 receives these lists and initiates interventions with the patients with the identified disease. These processes are described in more detail below. Such interventions may take the form of 1) specific case management, 2) novel interventions based on subgroup characteristics, 3) high risk intervention, 4) high (relative) cost intervention, or 5) plan modification all adhering, of course, to the best practice guidelines.

Referring to Figure 1B, the Risk Stratification (RS) process 140 is required to support the Disease Management system by providing the Intervention Management process 160 with a list of patients who are at-risk of an adverse health outcome for an identified disease. This list of patients is called the Intervention List.

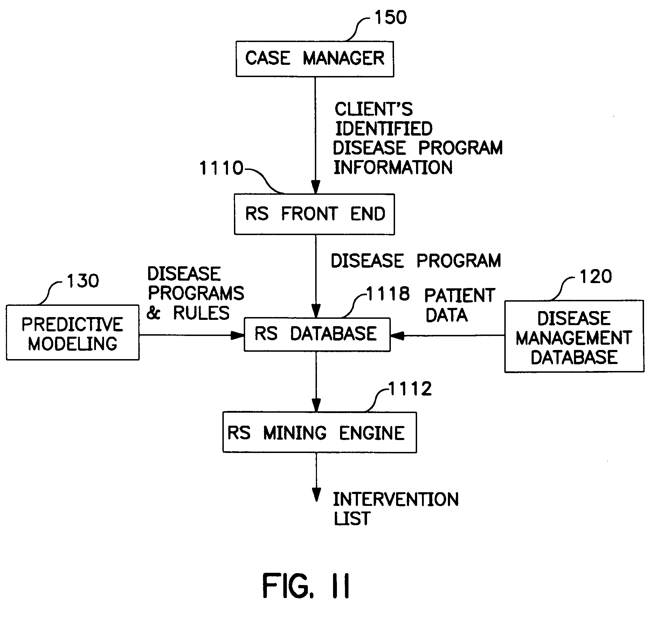

Figure 11 shows a high level flowchart showing the Risk Stratification process 140 including a RS Front End (FE) 1110 module, a RS Mining Engine (ME) 1112 module, and a RS Database 1118. These two modules collaborate to produce intervention lists from the RS Database 1118.

The RS Front End (FE) 1110 allows end users to enter all of the information necessary to maintain and run disease programs for clients.

The RS FE 1110 of the present invention is written using Delphi 2.0, which is a 32 bit software development tool. The RS FE 1110 stores client and disease parameters in Sybase System 11 running on a Windows NT or UNIX based server. The RS FE 1110 uses the Borland 32 Bit Sybase SQL Links database drivers. However, it is contemplated that the present invention can be practiced using any similar development and database tools and is not limited to this configuration.

The RS Mining Engine (ME) 1112 runs the scheduled client identified disease programs yielding intervention lists that are provided to the Intervention Management process 160 of Figure 1B. The RS ME 1112 is a batch/daemon process and follows this basic program logic:

    A. Run nightly (batch) or as a daemon process
    B. Determine what client identified disease programs need to run based on schedule and available data
    C. For every scheduled client disease program:

        a) Get disease program rule components.
        b) Get disease program parameters for each rule component.
        c) Validate that the necessary data streams ($R_x$, $M_x$ and Lab) exist for the identified disease program
        d) Initialize the scheduled client identified disease program
        e) Execute the scheduled client identified disease program
        f) Provide intervention lists to the Intervention Management process 160

    D. Terminate (batch) or set process to sleep (daemon)

The RS ME 1112 is written using Delphi 2.0, which is a 32 bit software development tool. The RS ME 1112 processes disease parameters provided by the RS FE 1110 combined with client pharmacy claims, medical claims and laboratory test information for specific disease programs producing specific intervention lists all of which are retrieved from or stored in a relational database. The RS ME 1112 utilizes the Sybase System 11 database running on a Windows NT or UNIX based server. The RS ME uses the Borland 32 Bit Sybase SQL Links database drivers. However, it is contemplated that the present invention can be practiced using any similar development and database tools and is not limited to this configuration.

The operation of the Risk Stratification process of Figure 11 is now described. End users, which may either be coupled to the Case Management process 150 of Figure 1B or another separate entity, provide end user identified disease program information to the RS FE 1110. The RS FE records information for the setup of new identified diseases, new disease programs, predictive models and rules, client specific parameters, disease specific rule parameters, and new clients; and the RS FE 1110 associates disease programs with clients, schedules disease programs, and runs informational reports. The RS FE 1110 records this information as a "disease program" in a format for use by the RS ME 1112.

The disease program is provided by the RS FE 1110 to the RS database 1118, and the RS database 1118 also receives predictive model and rule information from the Predictive Modeling process 130. Finally, the Disease Management database 120 provides patient medical information to the RS database 1118 for the RS ME 1112 when the RS ME 1112 applies the predictive model to the patient data. Finally, the RS ME 1112 receives the information contained in the RS Database 1118 as the RS ME executes the disease program data and applies the predictive model to the patient data.

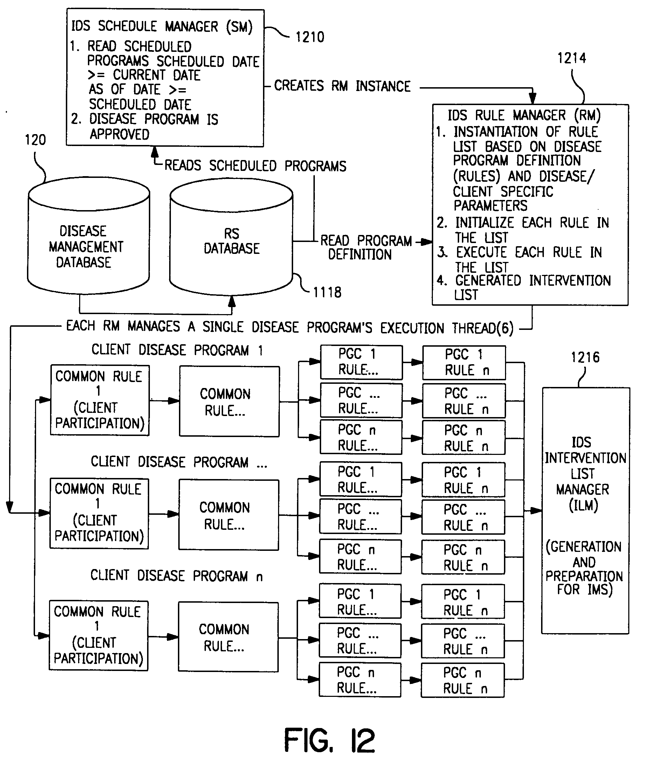

Figure 12 is a high level flowchart showing the RS ME 1112 of the Risk Stratification process of the present invention. The RS ME 1112, as shown in Figure 12, is composed of three major sub-systems: a RS Schedule Manager (SM) 1210, a RS Rule Manager (RM) 1214 and a RS Intervention List Manager (ILM) 1216. Each of the three sub-systems interacts with the RS Database 1118, which can be a subset of the Disease Management database 120 of Figure 1B, containing client and identified disease program analytic configurations.

The Disease Management database 120 is regularly updated with patient information (Member, Eligibility, Pharmacy ($R_x$) Claims, Medical ($M_x$) Claims and Clinical Laboratory (Lab) Claims) for each client. Consequently, the RS Database 1118 is also updated regularly with client and client member information. The RS ME 1112 gathers relevant client patient information from the Disease Management Database 120 to be processed by the disease program analytic rules. In the exemplary embodiment of the invention, all relational databases are SYBASE System 11.

The RS SM 1210 compiles a list of identified disease programs to execute by examining each enrolled client to see if the schedule time has arrived for the program to execute. Additionally, client disease programs must be approved for execution by the RS ME 1112 before they may be scheduled. Approval indicates that all client disease program parameters are entered and that the data entered has been validated by the RS FE 1110 and is ready to be processed in the RS ME 1112. Finally, the RS SM 1210 verifies that all required data streams are available. The RS ME 1112 may be a batch program that is executed periodically. For each identified disease program which is selected by the above logic, an RS RM object is created. RS RM objects are executed sequentially.

The RS RM 1216 then assembles the rules required to implement the specific identified disease program into an ordered sequence. These rules are described in detail subsequently, and are provided by the Predictive Modeling process 130 and the Case Manager 150. Each rule object is initialized with disease program and client specific rule arguments. Rules sequences desirably contain one or more Common rules and one or more sequence of rules called Patient Group Classifiers (PGCs). PGCs are used to stratify a targeted client patient population into specific groups for intervention or reporting based on specific criteria. All interventions and reporting is performed based on patient membership in one or more of the disease program PGCs.

Common rules are executed in the specified order prior to any PGCs. In general, rules are designated as common rules because they either prepare the environment for other rules (Client Participation, $R_x$ Claims, $M_x$ Claims, etc.) or they perform exclusions that reduce the overall patient set size prior to being acted upon by other complex rules (Patient Active, Patient Age, Patient Gender, etc.), thus improving overall performance. Patients who 'fail' the specified rules are removed from the patient set.

PGCs are executed in parallel with the rules in each PGC also being executed in parallel on the patient set provided by the common rules. PGC rules use a tally mechanism for each patient in the set to indicate passage or failure of the specified rule for that patient.

Upon completion of all PGCs the RS ILM 1216 scores each patient for membership in each PGC. The RS ILM 1216 then generates and stores intervention lists for later processing by the Intervention Management process 160.

The RS SM 1210 initially queries the RS Database 1118 at startup of batch process or periodically if running as a daemon to determine if the approved client identified disease programs scheduled run date has arrived and if all required client data streams are up to date. If all required data streams are available, a Rule Manager (RM) object is created for each client disease program.

Identified disease program attributes are stored in a table. One attribute is the approval status. Each identified disease program is desirably approved before it is scheduled. If any identified disease programs are scheduled, then the disease program approval may not be revoked.

Determining which programs require execution and when is accomplished via a schedule table which contains, among other things, a status and a scheduled run date. Once the scheduled date is reached the program is executed and the status is updated to running.

The RS RM 1214 is responsible for running and managing the results of a single disease program.

The rules are grouped according the Patient Group Classifier (PGC) that they are assigned to. First all the common rules (those without a PGC) are run. Then the rules for each PGC which exists in the disease program are run

The RS ILM 1216 evaluates each client disease program that successfully executed and compiles a listing in a intervention candidates table of the members selected by the program as belonging to each PGC within that program.

A member is included in a PGC if the member has not been deleted from the set by any common rule, and the member's output for each PGC rule matches the desired value (1 for non-negated rules and null for negated rules).

Members who are included in a PGC are populated into an interventions table, which can also be the intervention list. This table includes identifying information for the member selected, the program run, the PGC in which the member was included, and the physician which was identified if the Physician Identification Rule was used.

**Rules - General Classification**

A rule classified as a "Root Rule" indicates that the rule is required to run before all others and performs certain environment initializations for all other rules. Every identified disease program must have one and only one root rule. Currently, the only root rule is Client Participation.

A rule classified as a "Common Rule" indicates that the rule is eligible to be executed prior to any PGC. Members who 'fail' common rules are removed from the patient set. A rule can be simultaneously eligible to be executed as a common rule and a PGC rule.

A rule classified as a "PGC Rule" indicates that the rule is eligible to be executed after common rules in parallel. Members who 'pass' PGC rules are marked in a column specifically added for that rule in a table. A rule can be simultaneously eligible to be executed as a common rule and a PGC rule.

The rule "Creates Pharmacy Claims" creates a table for pharmacy claims. Every identified disease program that uses pharmacy claims for a data source desirably has a rule that performs this function prior to rules that use pharmacy claims.

The rule "Creates Medical Claims" creates a table for medical claims. Every identified disease program that uses medical claims desirably has a rule that performs this function prior to rules that use medical claims.

The rule "Creates Clinical Test Data" creates a table for clinical test data. Every disease program that uses laboratory claims desirably has a rule that performs this function prior to rules that use laboratory claims.

The rule "Uses Specialties" uses physician specialty information.

The rule "Uses Pharmacy Claims" uses the table containing pharmacy claims information.

The rule "Uses Medical Claims" uses the table containing medical claims information.

The rule "Uses Clinical Test Data" uses the table containing clinical test information.

All the rule objects in the RS ME 1112 are descended from a common ancestor which provides some basic functional structure shared by all rules

**Rules Selection Rules and Intervention Rules**

The present embodiment of the RS ME 1112 supports various selection and intervention rules:

1) Client Participation Rule

Identifies whether a patient is part of a group that has been enrolled into the disease management program. This rule will ensure that all patients considered by the following rules are part of a group that the client wishes to have participate in the program. This rule may also validate that the patient has the proper benefit structure to permit the disease program to function. Client Participation is currently the only root rule. It is desirably, therefore, the first rule in every disease program. It is always executed as a common rule.

2) $R_x$ Claim Rule

This rule selects all pharmacy claims data that is applicable to the execution of a single identified disease program. It identifies all pharmacy prescription claims selected for a specific drug group within a specified analytic time frame. The $R_x$ Claim rule is always a common rule. It is typically only run once in a given program.

3) Existence of a Specific Drug Rule

This rule identifies members with at least one claim for a drug in the specified drug group within the rule time frame. This rule may be run as either a common or a PGC rule.

4) Recurrent Patient Rule

This rule identifies whether a patient has a pattern of drug use which indicates the potential of multiple independent episodes (recurrence) of a disease. The rule will select patients with at least a certain number of discrete episodes of a particular drug therapy. This rule may be run as either a common or a PGC rule.

5) Stoppage in Current Therapy Rule

This rule identifies patients whose drug therapy for a particular drug group has been stopped. This is determined based on the last prescription for a drug in that drug group. This rule may be run as either a common or a PCG rule.

6) Patient Age Rule

This rule identifies patients whose ages fall within a specified target range. This rule may be run as either a common or a PGC rule.

7) Minimum Patient Eligibility Rule

This rule identifies whether a patient is eligible for medical and/or drug benefits for a specified continuous period of time. This rule may be run as either a common or a PGC rule.

8) Patient Active Rule

This rule verifies that a member is active and in a group which is included in the program at the time of intervention. This rule may be run as either a common or a PGC rule.

9) Average Puff Equivalence Rule

This rule identifies whether a member has the required average puff equivalence of drug therapy during a specified time frame. This rule may be run as either a common or a PGC rule.

10) Count of Occurrences Rule

This rule identifies whether a patient has a selected range of occurrences on different filled dates for a specified drug therapy. This rule may be run as either a common or a PGC rule.

11) Patient Gender Rule

This rule identifies members of a particular gender. This rule may be run as either a common or a PGC rule.

12) Dose Level Recurrence Rule

This rule identifies whether a patient has a pattern of drug use within a specified dose range which indicates the potential of multiple independent episodes (recurrence) of the disease at the same or similar severity. This rule may be run as either a common or a PGC rule.

13) Continuous Therapy at Required Dose Level Rule

This rules identifies patients who have continuous drug therapy within a specific dose range for a specified length of time. This rule may be run as either a common or a PGC rule.

14) Concurrent Therapy Rule

This rule identifies patients who have overlapping therapy of at least a given duration for the specified drug groups. This rule may be run as either a common or a PGC rule.

15) Dose Level Rule

This rule identifies patients who have $R_x$ Claims for a specified drug therapy within a specified dose level range. This rule may be run as either a common or a PGC rule.

16) Drug Usage Level Rule

This rule identifies members whose drug usage relative to expected values is within a specified range. Typically, this rule will be used to determine members who are non-compliant with a specified drug therapy. This rule may be run as either a common or a PGC rule.

17) Weighted Existence of Specific Drug Rule

This rule identifies members whose drug therapies fall within a designated risk score range. Each drug therapy is assigned a risk score and a member's drug history is assessed to determine his/her accumulated risk score. This rule may be run as either a common or a PGC rule.

18) Physician Identification Rule

This rule selects the specific Prescriber to send communication regarding a member who has been identified for intervention. This selection is based on the Pharmacy Claim data for that member and/or information about the member's primary care physician which may be found in the Member data in the Patient Data Repository 120. This rule may be run as either a common or a PGC rule.

19) All Member Rule

The All Member Rule selects all members present in the record set. This is used to support a PGC which contains all members selected by the common rules. This rule may also be used internally by the RS ME 1112 in order to support certain types of disease program optimization. This rule may only be used as a PGC rule.

Appendix VI includes a list and description of the selection rules as used in one embodiment of the invention. It should be apparent to those skilled in the art that these rules can be modified or deleted, and new rules created for a particular embodiment of the invention.

## Intervention Management Process

Once again referring to Figure 1B, the Risk Stratification process 140 outputs the Intervention List to the Intervention Management process 160 to initiate specific interventions. Interventions may include initial offerings, fully administered disease programs, forwarding educational materials, inbound or outbound telecommunications, faxes, Email or Voice Response interactions with member patients identified on the Intervention list. The Intervention Management process 160 provides the intervention information to the Intervention Records and Tracking process 170, which records the interventions to determine if proactive disease management services improve specific disease outcomes.

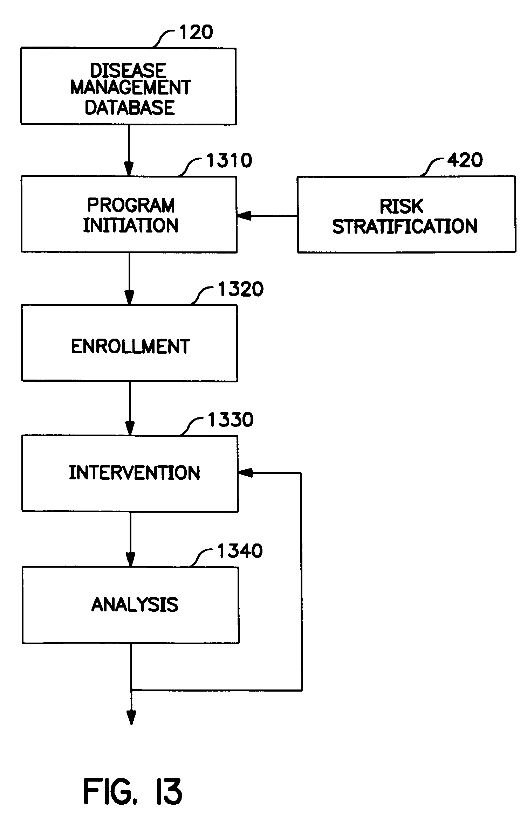

Figure 13 is a high level diagram of the Intervention Management process 160 of the present invention, and the intervention process, called an intervention program, is performed on an intervention list of client members having an identified disease. The Intervention Management process 160 shown in Figure 13 includes Program Initiation 1310, which starts the intervention program; Enrollment 1320, which enrolls identified patients into the intervention program; Intervention 1330, which initiates the intervention with the enrolled patient; and Analysis 1340, which analyzes the results of an intervention with a patient.

The Intervention Management process 160 is provided data by the Disease Management database 120 as well as the intervention list from the Risk Stratification process 140. This data feed or detection process has parameters that identify a specific patient meeting the conditions for participating in a disease program. This detection process provides a population for consideration in the specific disease program under the following conditions:

1) The Disease Management database 120 provides client's updated identified disease patient data to the intervention management system on a scheduled basis.
2) The Intervention Recording and Tracking process 170 passes intervention contact data back to the Disease Management database 120. This intervention data is stored there for use in the analytic process.
3) The Intervention Management process 160 detects, selects and passes new intervention data on "adds" which are defined as new enrollees, changes in disease detection, subsequent diagnosis or an individual enrollment request from an intervention manager.
4) The Intervention Recording and Tracking Process 170 revises patient data on those individuals previously selected for the program. Data revisions occur when personal or medical data changes. For example, additional medical or pharmacy claims are received or additional laboratory reports are secured.

Referring to Figure 13, the first step of the process is program initiation, step 1310. Program Initiation is a process where a disease program is initiated through the process of selection of a population of patients based on predefined criteria and the initial interventions are sent. Upon selection specific predefined program activities take place.

A sample initiation might be that 1) a letter is sent, on behalf of the patient, to their physician informing them of this patient's identification into the program, the disease protocols and the recommended actions from the physician. 2) Intervention Management data is passed from the Disease Management database 120 to the Intervention Management system 160 and is loaded. 3) An initial "contact segment" is added for the patient indicating the sending of the physician letter.

Another sample initiation might be: 1) a letter is sent to the patient, with a copy to their physician informing them of their inclusion in the disease program. 2) The patient may be requested to call into a Voice Response System to answer specific questions. 3) The contact is added and the responses analyzed for further processing.

The second step of the process is the Enrollment step 1320. In this step the Patients are enrolled into the program. Patients are enrolled into the Disease Management service through interfaces to the Intervention Management System. These interfaces can be through a Voice Response System, written letter return or a direct call. The enrollment process triggers the scheduling of an intervention event within the intervention management system.

The next step is the Intervention process 1330, which is the process of interceding with a physician and client for the purposes of: 1) ensuring compliance with a course of treatment, 2) providing disease educational material to both the patient and physician, 3) providing emergency assistance from a distance, 3) logging each and every intervention as a "contact" to provide assistance in determining program effectiveness and to establish a framework to make mid-course adjustments to the program, and 4) providing data back to the product managers on program effectiveness.

The last step is the analytic process 1340 which assimilates disease information for the purposes of determining disease management service success. Although the intervention management system does not produce the analytic reporting, critical information is passed back during this process to the Disease Management Database 120 for processing.

While the invention has been described in terms of an exemplary embodiment, it is contemplated that it may be practiced as outlined above with modifications that are within the scope of the following claims.

## Claims

1. A computer-implemented method for disease or condition intervention management using information about patients existing in at least one database, said method comprising the steps of:

a) processing, based on predetermined criteria, the patient information in the database to extract patient information for a group of patients relating to an identified disease or condition;

b) defining a predictive model, including:

 i) defining, using the information available in the database, a set of events or data relevant to the identified disease or condition;

 ii) converting the extracted patient information and the defined events or data into files comprising event-level information;

 iii) defining a time-window for providing a timeframe from which to judge whether specific ones of the defined events should be considered in subsequent processing;

 iv) identifying a set of variables as potential predictors;

 v) processing the event-level information, using the time-window and the set of variables, to generate an analysis file;

 vi) performing statistical analysis on the analysis file to generate the prediction model and a set of rules for use in identifying at-risk patients diagnosed with or who may develop the identified disease or condition, said prediction model and rules being a function of a subset of the set of variables;

c) applying the prediction model and the rules to the same or new set of event-level information to identify at-risk patients for the identified disease or condition, or to identify patients who may be at risk for developing the identified disease or condition;

d) preparing an intervention list from the identified at-risk patients and selecting, for at least one at risk patient, an intervention;

e) distributing or facilitating the distribution of the intervention to said patient; and optionally

f) recording and tracking an intervention result for each at-risk patient based on the respective selected intervention: and optionally

g) updating the historical data in at least one database with each intervention result corresponding to said database; and

h) repeating step b(ii); and

l) re-applying the prediction model and rules to the event-level information extracted from the data in the updated database.

2. A computer-implemented system for disease management using information about patients existing in a database, said system comprising:

a) processing means for processing, based on predetermined criteria, the patient information in the database to extract patient information for a group of patients having an identified disease or condition;

b) means for defining a predictive model, including:

 i) event definition means for defining, using the information available in the database, a set of events relevant to the identified disease or condition;

 ii) conversion means for converting the extracted patient information and the defined events into files containing event-level information;

 iii) means for defining a time window for providing a timeframe from which to judge whether specific ones of the defined events should be considered in subsequent processing;

 iv) means defining a set of variables as potential predictors;

 v) means for processing the event-level information, using the time window and the set of variables, to generate an analysis file;

 vi) means for performing statistical analysis on the analysis file to generate the prediction model and a set of rules for use in identifying at-risk patients diagnosed with the identified disease, said prediction model and rules being a function of a subset of the set of variables;

c) means for applying the prediction model and the rules to the same or new set of event-level information to identify at-risk patients for the identified disease or condition;

d) means for forming an intervention list from the identified at-risk patients and selecting, for at least one at risk patient, an intervention;

e) means for distributing or facilitating the distribution of the intervention to said patient; and optionally

f) means for recording and tracking an intervention result for each at-risk patient based on the respective selected intervention; and

g) means for updating the historical data in at least one database with each intervention result corresponding to said database or creating a mirror database using the data obtained in step f); and

h) means for repeating step b(i); and

i) means for re-applying the prediction model and rules to the event-level information extracted from the data in the updated database.

3. A process for preparing a health intervention product from patient information in a computer database said process comprising:

a) using a computer for extracting and processing, based on predetermined criteria, the patient information in the database to obtain a data file of patient information for a group of patients having an identified disease or condition;

b) programming a predictive model into a computer wherein the model constructed includes the steps of:

i) defining, using the information available in the database, a set of events relevant to the identified disease or condition;

ii) converting the extracted patient information and the defined events into files containing event-level information;

iii) applying a time window for providing a timeframe from which to judge whether specific ones of the defined events should be considered in subsequent processing;

iv) entering a set of variables as potential predictors;

v) generating an analysis file by processing the event-level information, using the time window and the set of variables;

vi) performing statistical analysis on the analysis file to generate the prediction model and a set of rules for use in identifying at-risk patients diagnosed with the identified disease or condition, said prediction model and rules being a function of a subset of the set of variables; then
on a computer:

c) running the prediction model and the rules against the same or new set of event-level information to identify at-risk patients for the identified disease or condition;

d) outputting an intervention list from the identified at-risk patients and selecting, for at least one at risk patient, an intervention;

e) distributing the intervention to said patient; and optionally

f) recording and tracking an intervention result for each at-risk patient based on the respective selected intervention; and

g) updating the historical data in at least one database or creating a new database with each intervention result corresponding to said database; and

h) re-running step b(i); and

i) re-running the prediction model and rules against the event-level information extracted from the data in the database created in step g; and optionally

j) outputting an intervention list obtained by re-running the prediction model and the rules against the database created in step g.

4. A health intervention product made by the process of:

a) using a computer for extracting and processing, based on predetermined criteria, the patient information in the database to obtain a data file of patient information for a group of patients having an identified disease or condition;

b) programming a predictive model into a computer wherein the model constructed includes the steps of:

i) defining, using the information available in the database, a set of events relevant to the identified disease or condition;

ii) converting the extracted patient information and the defined events into files containing event-level information;

iii) applying a time window for providing a timeframe from which to judge whether specific ones of the defined events should be considered in subsequent processing;

iv) entering a set of variables as potential predictors;

v) generating an analysis file by processing the event-level information, using the time window and the set of variables;

vi) performing statistical analysis on the analysis file to generate the prediction model and a set of rules for use in identifying at-risk patients diagnosed with the identified disease or condition, said prediction model and rules being a function of a subset of the set of variables; then

on a computer:

c) running the prediction model and the rules against the same or new set of event-level information to identify at-risk patients for the identified disease or condition;

d) outputting in hard copy or machine-readable form an intervention list from the identified at-risk patients and selecting, for at least one at risk patient, an intervention;

e) distributing the intervention to said patient; and optionally

f) recording and tracking an intervention result for each at-risk patient based on the respective selected intervention; and

g) updating the historical data in at least one database or creating a new database with each intervention result corresponding to said database; and

h) re-running step b(i); and

i) re-running the prediction model and rules against the event-level information extracted from the data in the database created in step g; and optionally

j) outputting an intervention list obtained by re-running the prediction model and the rules against the database created in step g.

FIG. IA

FIG. IB

FIG. 2A

RESOURCES

**Rx**

NAME
DATE PRESCRIBED
DRUG CODE
AMT DISPENSED
PHYSICIAN NAME
AMT CLAIMED
AMT R-REIMBURSED
........

**DOCTOR**

NAME
PHYSICIAN
DIAGNOSIS-ICD-9
PROCEDURES
DATE
AMT CLAIMED
........

**HOSPITAL**

NAME
HOSPITAL-SITE CODE
PHYSICIAN
DIAGNOSIS
DATE
PROCEDURES
LENGTH OF STAY
........

FIG. 2B

FIG. 3

EP 0 917 078 A1

EP 0 917 078 A1

FIG. 4

## RESEARCH DATA BASE (RDB)

SAS FORMAT

| Rx | Dr | HL |
|---|---|---|
| ID, DATE DRUG... | ID, ICD, DATE... | ID, ICD, HOSP... |
| CLAIM 1 | CLAIM 1 | CLAIM 1 |
| . | . | . |
| . | . | . |
| . | . | . |
| CLAIM X | CLAIM X | CLAIM X |

FIG. 5

EP 0 917 078 A1

250

```
┌─────────────────────┐
│  RESEARCH DATABASE  │
└─────────────────────┘
```

610          SAMPLE PATIENT DATA

```
┌─────────────────────┐
│  EXTRACT DISEASE    │
│ SAMPLE PATIENT DATA │
└─────────────────────┘
```

620

```
┌─────────────────────┐
│  QUALITY  CONTROL   │
│     (OPTIONAL)      │
└─────────────────────┘
```

630

```
        IS
       DATA
   STATISTICALLY        NO
      VALID?
```

YES          640

```
┌─────────────────────┐
│   CONVERT CLAIMS    │
│  LEVEL DATA TO EVENT│
│     LEVEL DATA      │
└─────────────────────┘
```

650

```
┌─────────────────────┐
│   PROCESS EVENT     │◄──── TIME FRAME
│  LEVEL FILES INTO   │
│    ANALYSIS FILES   │◄──── VARIABLES
└─────────────────────┘
```

660

```
┌─────────────────────┐
│  PROCESS ANALYSIS   │
│    FILE USING       │
│  STATISTICAL TECH.  │
└─────────────────────┘
```

IDENTIFIED DISEASE
PREDICTION MODEL

FIG. 6

| | EVENT | EVENT DATE | NUMBER | COST | CATEGORY INDICATOR |
|---|---|---|---|---|---|
| 1 | HOSPITALIZATION FOR DEPRESSION | DATE | LOS | $ | SEVERITY |
| 2 | EMERGENCY ROOM FOR DEPRESSION | DATE | BLANK | $ | BLANK |
| 3 | DOCTOR (OUTPATIENT) VISIT FOR DEPRESSION | DATE | BLANK | $ | SPECIALIST |
| 4 | PRESCRIPTION FOR SSRI | DATE | DAYS SUPPLY | $ | THERAPY CLASS |
| 5 | PRESCRIPTION FOR TCA | DATE | DAYS SUPPLY | $ | THERAPY CLASS |
| 6 | PRESCRIPTION FOR OTHER NEUROACTIVE DRUG | DATE | DAYS SUPPLY | $ | SUB-CLASS |
| 7 | PROCEDURE FOR DEPRESSION | DATE | BLANK | $ | SUB-CLASS |
| 8 | HOSPITALIZATION NOT FOR DEPRESSION | DATE | LOS | $ | SEVERITY |
| 9 | EMERGENCY ROOM NOT FOR DEPRESSION | DATE OF FIRST | NUMBER IN MONTH | $ | |
| 10 | DOCTOR (OUTPATIENT) VISIT NOT FOR DEPRESSION | DATE OF FIRST | NUMBER IN MONTH | $ | |
| 11 | PRESCRIPTION FOR POSSIBLY RELATED DRUGS | DATE OF FIRST | NUMBER IN MONTH | $ | |
| 12 | PRESCRIPTION FOR NON-DEPRESSION DRUGS | DATE OF FIRST | NUMBER IN MONTH | $ | |
| 13 | PROCEDURE NOT FOR DEPRESSION | DATE OF FIRST | NUMBER IN MONTH | $ | SEVERITY INDICATOR |

## FIG. 7A

| EVENT | EVENT DATE | NUMBER | COST | CATEGORY INDICATOR |
|---|---|---|---|---|
| 1. CHF ER | DATE | NA | $ | NA |
| 2. CHF HOSPITALIZATION | DATE | LOS | $ | NA |
| 3. CHF OFFICE VISIT | DATE | NA | $ | NA |
| 4. | | | | |

## FIG. 7B

| | EVENT | EVENT DATE | NUMBER | COST | CATEGORY INDICATOR |
|---|---|---|---|---|---|
| 5. | IHD HOSPITALIZATION | DATE | LOS | $ | SUBCLASS |
| 6. | IHD OFFICE VISIT | DATE | NA | $ | SUBCLASS |
| 7. | DIABETES ER | DATE | NA | $ | NA |
| 8. | DIABETES HOSPITALIZATION | DATE | LOS | $ | NA |
| 9. | DIABETES OFFICE VISIT | DATE | NA | $ | NA |
| 10. | DYSRHYTHMIA ER | DATE | NA | $ | NA |
| 11. | DYSRHYTHMIA HOSPITALIZATION | DATE | LOS | $ | NA |
| 12. | DYSRHYTHMIA OFFICE VISIT | DATE | NA | $ | NA |
| 13. | HYPERTENSION ER | DATE | NA | $ | NA |
| 14. | HYPERTENSION HOSPITALIZATION | DATE | LOS | $ | NA |
| 15. | HYPERTENSION OFFICE VISIT | DATE | NA | $ | NA |
| 16. | LIFESTYLE ER | DATE | NA | $ | NA |
| 17. | LIFESTYLE HOSPITALIZATION | DATE | LOS | $ | NA |
| 18. | LIFESTYLE OFFICE VISIT | DATE | NA | $ | NA |
| 19. | OTHER HRT DZ ER | DATE | NA | $ | NA |
| 20. | OTHER HRT DZ HOSPITALIZATION | DATE | LOS | $ | NA |
| 21. | OTHER HRT DZ OFFICE VISIT | DATE | NA | $ | NA |
| 22. | RESPIRATORY ER | DATE | NA | $ | NA |
| 23. | RESPIRATORY HOSPITALIZATION | DATE | LOS | $ | NA |
| 24. | RESPIRATORY OFFICE VISIT | DATE | NA | $ | NA |
| 25. | THYROTOXICOSIS ER | DATE | NA | $ | NA |
| 26. | THYROTOXICOSIS HOSPITALIZATION | DATE | LOS | $ | NA |
| 27. | THYROTOXICOSIS OFFICE VISIT | DATE | NA | $ | NA |
| 28. | PULMONARY EMBOLISM ER | DATE | NA | $ | NA |
| 29. | PULMONARY EMBOLISM HOSP. | DATE | LOS | $ | NA |
| 30. | PULMONARY EMBOLISM OFFICE | DATE | NA | $ | NA |
| 31. | ANEMIA ER | DATE | NA | $ | NA |
| 32. | ANEMIA HOSPITALIZATION | DATE | LOS | $ | NA |
| 33. | ANEMIA OFFICE VISIT | DATE | NA | $ | NA |
| 34. | INFECTION ER | DATE | NA | $ | NA |
| 35. | INFECTION HOSPITALIZATION | DATE | LOS | $ | NA |
| 36. | INFECTION OFFICE VISIT | DATE | NA | $ | NA |
| 37. | OTHER ER | DATE OF FIRST | # ER IN MONTH | $ | NA |
| 38. | OTHER HOSPITALIZATION | DATE OF FIRST | # HOSP IN MONTH | $ | NA |
| 39. | OTHER OFFICE VISIT | DATE OF FIRST | # OV IN MONTH | $ | NA |
| 40. | MISCELLANEOUS MEDICAL EVENT | DATE OF FIRST | # IN MONTH | $ | # OF CV IN MONTH |
| 41. | ROUTINE CV PROCEDURES | DATE OF FIRST | # ON EVENT DATE | $ | NA |
| 42. | INTERMEDIATE CV PROCEDURES | DATE OF FIRST | # ON EVENT DATE | $ | NA |
| 43. | CRITICAL CV PROCEDURES | DATE OF FIRST | # ON EVENT DATE | $ | NA |
| 44. | CV SURGERY | DATE OF FIRST | # ON EVENT DATE | $ | NA |
| 45. | Rx FOR ACE INHIBITOR THERAPY | DATE OF FIRST | DAYS SUPPLY | $ | # RX IN MONTH |
| 46. | Rx FOR LOOP DIURETIC THERAPY | DATE OF FIRST | DAYS SUPPLY | $ | # RX IN MONTH |
| 47. | Rx FOR OTHER DIURETIC THERAPY | DATE OF FIRST | DAYS SUPPLY | $ | # RX IN MONTH |
| 48. | Rx FOR DIGOXIN THERAPY | DATE OF FIRST | DAYS SUPPLY | $ | # RX IN MONTH |
| 49. | Rx FOR BETA BLOCKER THERAPY | DATE OF FIRST | DAYS SUPPLY | $ | # RX IN MONTH |
| 50. | Rx FOR CA CHANNEL BLOCKER TX | DATE OF FIRST | DAYS SUPPLY | $ | # RX IN MONTH |
| 51. | Rx FOR OTHER CV DRUG | DATE OF FIRST | DAYS SUPPLY | $ | # RX IN MONTH |
| 52. | Rx FOR NON-CV DRUG | DATE OF FIRST | DAYS SUPPLY | $ | # RX IN MONTH |
| 53. | Rx FOR Na/H20 DRUG | DATE OF FIRST | DAYS SUPPLY | $ | # RX IN MONTH |

## FIG. 7C

ANALYSIS FILE

VARIABLES-CREATED FROM CLAIMS DATA ELEMENTS.
$X_1$ $X_2$ • • • $X_\eta$

MEMBER 1

MEMBER X

FIG. 8

FIG 9

TODAY

PREDICTION WINDOW

910

INDEX TIME

B

C

912

EVENTS WINDOW

A

MODEL—SCHEME 1

HOSPITAL vs. HOSPITAL

CASE DEFINITION

TIME

| | | | |
|---|---|---|---|
| −12 MONTHS | −6 MONTHS | 0 | +6 MONTHS |

$$\text{EVENT X} \quad \frac{1}{e-\text{TIME}}$$

## FIG. IOA

EP 0 917 078 A1

MODEL-SCHEME 2

CASE DEFINITION

LAST HOSPITAL

TIME

| |-----------------------|-----------------------|-------------|

-12 MONTHS          -6 MONTHS          0        3 MONTHS

$$\longleftarrow \text{EVENT X} \quad \frac{1}{e-\text{TIME}}$$

FIG. 10B

EP 0 917 078 A1

FIG. II

IDS SCHEDULE MANAGER (SM) — 1210

1. READ SCHEDULED PROGRAMS SCHEDULED DATE >= CURRENT DATE AS OF DATE >= SCHEDULED DATE
2. DISEASE PROGRAM IS APPROVED

— CREATES RM INSTANCE — 1214

IDS RULE MANAGER (RM)

1. INSTANTIATION OF RULE LIST BASED ON DISEASE PROGRAM DEFINITION (RULES) AND DISEASE/CLIENT SPECIFIC PARAMETERS
2. INITIALIZE EACH RULE IN THE LIST
3. EXECUTE EACH RULE IN THE LIST
4. GENERATED INTERVENTION LIST

READS SCHEDULED PROGRAMS

120

DISEASE MANAGEMENT DATABASE

RS DATABASE

READ PROGRAM DEFINITION

1118

EACH RM MANAGES A SINGLE DISEASE PROGRAM'S EXECUTION THREAD(6)

CLIENT DISEASE PROGRAM 1

COMMON RULE 1 (CLIENT PARTICIPATION)

COMMON RULE...

PGC 1 RULE...  |  PGC 1 RULE n
PGC ... RULE...  |  PGC ... RULE n
PGC n RULE...  |  PGC n RULE n

CLIENT DISEASE PROGRAM ...

COMMON RULE 1 (CLIENT PARTICIPATION)

COMMON RULE...

PGC 1 RULE...  |  PGC 1 RULE n
PGC ... RULE...  |  PGC ... RULE n
PGC n RULE...  |  PGC n RULE n

CLIENT DISEASE PROGRAM n

COMMON RULE 1 (CLIENT PARTICIPATION)

COMMON RULE...

PGC 1 RULE...  |  PGC 1 RULE n
PGC ... RULE...  |  PGC ... RULE n
PGC n RULE...  |  PGC n RULE n

1216

IDS INTERVENTION LIST MANAGER (ILM)

(GENERATION AND PREPARATION FOR IMS)

FIG. 12

FIG. 13

## EP 0 917 078 A1

European Patent
Office

**EUROPEAN SEARCH REPORT**

Application Number

EP 97 30 8240

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DYNE VAN M M ET AL: "USING MACHINE LEARNING AND EXPERT SYSTEMS TO PREDICT PRETERM DELIVERY IN PREGNANT WOMEN" PROCEEDINGS OF THE CONFERENCE ON ARTIFICIAL INTELLIGENCE FOR APPLICATIONS, 1 March 1994, SAN ANTONIO, pages 344-350, XP000479468 * the whole document * | 1-4 | G06F19/00 G06F9/44 |
| Y | GB 2 310 060 A (SMITHKLINE BEECHAM CORPORATION) * page 2, line 23 - page 3, line 23 * | 1-4 | |
| Y | STEFANELLI M ET AL: "KNOWLEDGE ACQUISITION BASED ON AN EPISTEMOLOGICAL MODEL OF MEDICAL REASONING" PROCEEDINGS OF THE ANNUAL INTERNATIONAL CONFERENCE OF THE IEEE ENGINEERING IN MEDICINE AND BIOLOGY SOCIETY, vol. 14 PART 3, 29 October 1992, PARIS, pages 880-882, XP000480668 * the whole document * | 1-4 | |

TECHNICAL FIELDS SEARCHED (Int.Cl.6)

G06F

The present search report has been drawn up for all claims

| Place of search | Date of completion of the search | Examiner |
|---|---|---|
| BERLIN | 5 March 1998 | Nicholls, J |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.** EP 97 30 8240

This annex lists the patent family members relating to the patent documents cited in the above-mentioned European search report.
The members are as contained in the European Patent Office EDP file on
The European Patent Office is in no way liable for these particulars which are merely given for the purpose of information.

05-03-1998

| Patent document cited in search report | Publication date | Patent family member(s) | Publication date |
|---|---|---|---|
| GB 2310060 A | 13-08-97 | AU 1262997 A<br>JP 10021298 A<br>EP 0813155 A | 21-08-97<br>23-01-98<br>17-12-97 |